Europäisches Patentamt

(19) European Patent Office

Office européen des brevets

(11) Publication number: **0 229 161 B1**

(12) **EUROPEAN PATENT SPECIFICATION**

(45) Date of publication of patent specification:
29.05.91 Bulletin 91/22

(51) Int. Cl.⁵: **C12P 13/22, C12N 15/52, C12N 1/00**

(21) Application number: 86904567.4

(22) Date of filing: 24.06.86

(86) International application number:
PCT/US86/01353

(87) International publication number:
WO 87/00202 15.01.87 Gazette 87/01

(54) COMPOSITE PLASMIDS FOR AMINO ACID SYNTHESIS.

(30) Priority: 24.06.85 US 747732

(43) Date of publication of application:
22.07.87 Bulletin 87/30

(45) Publication of the grant of the patent:
29.05.91 Bulletin 91/22

(84) Designated Contracting States:
DE FR GB IT

(56) References cited:
EP-A- 0 077 196
EP-A- 0 145 156
EP-A- 0 190 921
FR-A- 2 486 961
US-A- 4 371 614
BIOCHIMICA & BIOPHYSICA ACTA, vol. 717,
1982, pages 6-11, Elsevier Biomedical Press,
Amsterdam, NL; S.B. BHOSALE et al.:
"Production of chorismate mutase-prephe-
nate dehydrogenase by a strain of
Escherichia coli carrying a multicopy, tyrA
plasmid"
GENE, vol. 33, May 1985, pages 323-331,
Elsevier Science Publishers, Amsterdam, NL;
W.D. DAVIES et al.: "Cloning of aroG, the gene
coding for phospho-2-keto-3-deoxy-heptonate
aldolase(phe), in Escherichia coli K-12, and
subcloning of the aroG promoter and operator
in a promoter-detecting plasmid"
GENE, vol. 36, September 1985, pages
131-141, Elsevier Science Publishers, Amster-
dam, NL; HONIGMAN et al.: "Plasmid vectors
designed for the analysis of transcription ter-
mination signals"

(56) References cited:
K. AIDA et al.: "Biotechnology of amino acid
production", Progress in Industrial Microbiol-
ogy, vol. 24, 1986, pages 24-26,34,35,188-206,
Elsevier, Amsterdam, NL; T. BEPPU:
"Application of recombinant DNA technology
of breeding of amino acid-producing strains"
Biochimica et Biophysica Acta, vol. 228. Is-
sued January 1982 (Amsterdam, NL); Bhosale
et al:"Production of chorismate mutase-
prephenate dehydrogenase by a strain of
Escherichia coli carrying a multicopy tyrA
plasmid", pp. 6-11
Gene, vol. 33, issued May 1985 /AmsterdaM,
NL); Davies et al:"Cloning of aroG, the gene
coding for phospho-2-keto-3-deoxy-heptonate
aldolase (phe) IN Escherichia coli K-12, and
subcloning of the aroG promoter and operator
in a promoter detecting plasmid", pp. 323-331
Gene, vol. 36, issued September 1985 (Amster-
dam, NL); Honigman et al:"Plasmid vectors
designed for the analysis of transcription ter-
mination signals, pp. 131-141

(73) Proprietor: THE NUTRASWEET COMPANY (a
Delaware corporation)
1751 Lake Cook Road
Deerfield Illinois 60015 (US)

(72) Inventor: EDWARDS, Mark, Richard
4 Gladstone Rise
High Wycombe (GB)
Inventor: TAYLOR, Paul, Phillip
4 Park Street
Thame (GB)
Inventor: HUNTER, Michael, George
8 Tamar Close
Aylesbury (GB)
Inventor: FOTHERINGHAM, Ian, Graham
101 A London Rd.
Headington, Oxford (GB)

(74) Representative: Froud, Clive et al
ELKINGTON AND FIFE Beacon House 113
Kingsway
London WC2B 6PP (GB)

## Description

## BACKGROUND OF THE INVENTION

Described are methods for the production of composite plasmids containing multiple genes that have been individually mutated then assembled into transcriptional units. These composite plasmids are useful in the production of amino acids and other metabolites and in screening for superior host cells.

Bacterial fermentations are extensively employed for the industrial production of both primary metabolites such as amino acids and secondary metabolites such as antibiotics. The production of these metabolites is an industrial process of great economic significance.

Naturally occurring microorganisms do not in general overproduce any metabolites since the relevant biosynthetic pathways are tightly regulated to avoid any waste of resources. The development of industrially useful production strains requires an extensive program of mutagenesis followed by selection of improved strains. Such a selection process may become extremely laborious in the later stages of strain development and even involve the screening of individual isolates by fermentation.

Recombinant DNA technology has greatly facilitated strain development since it is now possible to isolate the important genes in a biosynthetic pathway and to deregulate them in vitro. In addition, the cloning of genes onto multicopy plasmids is an efficient way of boosting the expression of a gene and hence the level of the relevant protein within the cell.

Many biosynthetic pathways are complex, however, involving upwards of ten individual steps. In such cases the choice of genes and their organization on a vector becomes of prime importance when considering a recombinant DNA approach to strain development. This invention describes a process for organizing the genes from a biosynthetic pathway on a vector in a manner which maximizes the utility of the recombinant DNA approach to strain optimization and thereby greatly accelerates strain development. The products of this process are termed composite plasmids.

Plasmids containing individual genes or natural operons have been described, for example in L-phenylalanine production FR2486961-A, EP85958-A, GB2053906A, EP77196-A ; L-proline production GB2075056-A, EP85958 A, in interferon production, ED126338-A ; in tryptophan production, J59125892-A, EP80378A, J57080398-A, US4, 371, 614, EP124048A. These references do not disclose the composite plasmids described in this document. An amino acid Biosynthesis overview is described in the text "Amino Acids : Biosynthesis and Genetic Regulation", edited by K.M. Herrmann and R.L. Somerville, published by Addison Wesley Pub. Co. (1983).

A review of recombinant DNA procedures can be found in "Molecular Cloning : A Laboratory Manual" by T. Maniatis, E.T. Fritsch and J. Sambrook, published by Cold Spring Harbor Laboratory, NY (1982). U.S. Patent 4, 514, 502 describes plasmids containing drug resistance genes and a drive-unit region from a Coryneform glutamic acid-producing bacteria capable of propagating in E.coli or B.subtilis.

## SUMMARY OF THE INVENTION

As a composition of matter, a composite plasmid comprising :

(a) a first DNA segment containing a replicon covalently joined to a second DNA segment containing one or more transcriptional units ; (b) if only one transcriptional unit, then containing two or more feedback resistant genes coding for two or more enzymes active in the synthesis of an amino acid ; (c) if two or more transcriptional units, then a first transcriptional unit containing one or more feedback resistant genes coding for one or more enzymes active in the synthesis of an amino acid and one or more additional transcriptional units containing one or more genes coding for enzymes useful in the production of an amino acid.

Among the amino acids preferred are aromatic amino acids, particularly phenylalanine, tryptophan and tyrosine.

A preferred embodiment of the invention is a composite plasmid wherein an additional transcriptional unit contains a gene for a catabolic enzyme or a transport protein. A more preferred embodiment is a composite plasmid containing 2 or more feedback resistant genes selected from mutants of the following genes : pheA, aroF, tyrA, pheR, tyrR, tyrB, aroL, aroH, aroG, trpE, aspC, or trp BAD or C. Composite plasmids are capable of replication in procaryotic or eukaryotic cells, given transcription units specific for each cell type.

One object of the invention is a transcriptional unit containing a catabolic enzyme or a permease useful in amino acid synthesis. A preferred embodiment of the invention is the utilization of an amylase on one of the transcriptional units of the composite plasmid.

Another object of the invention is a method of screening for microorganisms useful in amino acid production wherein a composite plasmid containing a gene coding for a feedback resistant enzyme catalyzing a rate-limiting step in amino acid production is used to transform a cell to be screened, then quantitating the production of the desired amino acid.

The composite plasmids of the invention can be used to screen any Gram-negative organism. Similarly, any Gram negative microorganism can be utilized in amino acid production using the composite plasmids. The composite plasmids exemplified can be used in Gram-negative microorganisms to produce aromatic amino acids, particularly L-phenylalanine and L-tyrosine. Among the Gram negative microorganisms that can be used are the following genera : Neisseria, Veillonella, Brucella, Bordetella, Pasteurella, Haemophilus, Escherichia, Erwinia, Shigella, Salmonella, Proteus, Yersinia, Enterobacter, Serratia, Azotobacter, Rhizobium, Nitro-somonas, Nitrobacter, Thiobacillus, Pseudomonas, Acetobacter, Photobacterium, Zymomonas, Aeromonas, Vibrio, Desulfovibrio, and Spirillum.

Also described is a method for producing a composite plasmid by mutating individual genes to a feedback resistant condition such that the enzymes produced remain active in amino acid synthesis even in the presence of surplus amino acid product, then connecting the mutated genes to a transcriptional unit.

Preferred embodiments of composite plasmids are pME202, pME219, pME214, and pPT112.

## BRIEF DESCRIPTION OF THE FIGURES

Figure 1 – Schematic of aromatic amino acid biosynthetic pathway

Figure 2 – Growth curves of HW77/pPT112 on glucose and starch

Figure 3 – Fermentation of HW77/pPT112 on soluble starch

Figure 4 – Screening strains for phenylalanine production using composite plasmids for L-phenylalanine production (pME202) with zone size measured after 48 hr.

Figure 5 – Comparison of phenylalanine production by HW77 and HW77/pME202 during fermentation

## DESCRIPTION OF THE PREFERRED EMBODIMENTS

One object of the invention is to combine on a suitable vector the genes coding for the enzymes that are of greatest importance in influencing the flux of carbon down the biosynthetic pathway of interest. These enzymes are usually the points in the pathway at which regulation principally occurs. In addition to these regulated genes, it is also necessary to include the genes for other enzymes which modulate the regulated steps. These genes usually are in the biosynthetic pathway of interest but they also occur within intermediary metabolism and consequently affect the supply of precursors to the pathway. Other genes may be of more indirect importance. For example, it may be necessary to include : the genes of pathway regulators, either to enhance the flux of interest or to decrease flux through an undesired competing pathway ; genes that influence permeability of the cells, to either substrates or products ; genes that allow growth of the organism on a more desirable substrate ; genes involved in regulating the cloned genes in such a manner that they can be turned on or off in a manner that facilitates the fermentation ; genes that can be used to influence the stability of the plasmid or to select for the plasmid.

The genes of interest for a composite plasmid may optimally be organized as a synthetic or semi-synthetic operon in which all the genes are transcribed from a promoter of the desired regulatory characteristics (see chart 1). Ideally the genes are modified such that they are simply assembled by the use of intervening restriction sites. Use was made of the restriction enzyme BamHI which allows for overlap of both the useful restriction site and a functional ribosome binding site. Finally, it was best to include a transcriptional terminator sequence downstream of the final gene in the operon to limit the extent of the transcript. The whole operon ideally is flanked by restriction sites so that it can be treated as a "cassette", and contain as little redundant DNA as possible to minimize its size. Such an operon was constructed by conventional recombinant DNA techniques, involving initial cloning of the genes followed by the determination of their DNA sequence, directed mutagenesis to introduce the desired restriction sites, and construction of optimal linkers, promoters and terminators by the use of synthetic oligonucleotides. These recombinant DNA techniques are well known to those skilled in the art of genetic engineering.

A transcription unit is defined schematically in Chart 1. It contains assembled components not found together in nature and consists of a regulatory region containing a promoter/operator region, one or more genes mutated to feedback resistance, one or more restriction sites situated between components of the transcription unit and a transcription terminator after the last gene.

## Chart 1 General Structure of an Operon for the Optimum

### Organization of Genes

$$R_1 \quad R_2 \qquad\qquad R_3 \qquad\qquad R_4 \qquad\qquad R_{n+2}$$

/P/O/****gene 1****/__/****gene 2****/__/****gene_n****/T/

p = promoter
o = operator (may overlap promoter)
Site of desired transcriptional regulation (positive or negative)
(gene) 1 – (gene) n = genes of interest to relevant biosynthetic pathway.
T = Transcriptional terminator.
$R_1$-$R_n$ = Restriction sites to facilitate construction and/or manipulation of operon.

## ADVANTAGES OF COMPOSITE PLASMIDS

A composite plasmid is exceedingly versatile and increases the flexibility of the recombinant DNA approach to strain development in the following ways.

1. If the cloned genes for the regulated steps in the pathway have not already been mutated to yield feed-back inhibition resistant (FBI^R) derivatives then mutagenesis of the plasmid and screening for producers will quickly yield the desired mutants. This method has the advantage that the recipient background is not subject to mutagenesis and therefore the accumulation of unknown and possibly deleterious mutations that occurs with conventional strain development is avoided.

2. The operon can be simply switched between vectors with different copy numbers to assess the level of expression for optimum productivity.

3. Similarly, the operon can be switched between different vectors to optimize stability. If necessary the operon can be simply reintegrated into the host genetic background.

4. The level of expression can be varied at a given copy number by simple modification to one promoter.

5. The ratio of the different gene products can be finely tuned by altering the efficiency of the ribosome binding sites (RBS). This in turn is greatly facilitated by the presence of restriction sites within the ribosome binding sequences. If necessary a wide disparity in required levels of expression could be accommodated by the use of an attenuator or by splitting the construct into two operons with promoters of different strengths.

6. Since transformation with a plasmid carrying such a construct effectively deregulates a strain immediately it is a very rapid method of screening for undefined differences between strains that influence productivity and thus choosing the best background for a producing strain.

7. Since such a plasmid can embody all that is known about a pathway it can be used in conjunction with mutagenesis to screen for mutants of a particular strain that offer increased productivity. A strain can be mutagenized followed by transformation and then screened for even more productive strains. This avoids firstly the many rounds of mutagenesis that would be required to deregulate the pathway to the same extent as the plasmid and secondly the potentially deleterious effects of mutagenesis on the cloned genes.

8. The operon can be cloned into plasmids with different or extended host ranges to allow for production in different species or genera. The plasmid will be relatively simple to optimize in these back-grounds by virtue of its single promoter and the presence of restriction sites in the RBS sequences.

9. Additional genes of importance can readily be incorporated as the requirements of the fermentation alter or as understanding if the system evolves.

10. Since the complexity of competing transcription/expression is avoided the construction and optimization of plasmids carrying many genes involved with the biosynthesis of a product is made much easier.

DESCRIPTION OF THE EXAMPLES

As examples of the application of this invention we now describe how the biosynthesis of the amino acids phenylalanine and tyrosine can be significantly deregulated and how synthetic operon plasmids can be usefully employed in the development of strains that produce aromatic amino acids, such a L-phenylalanine or L-tyrosine.

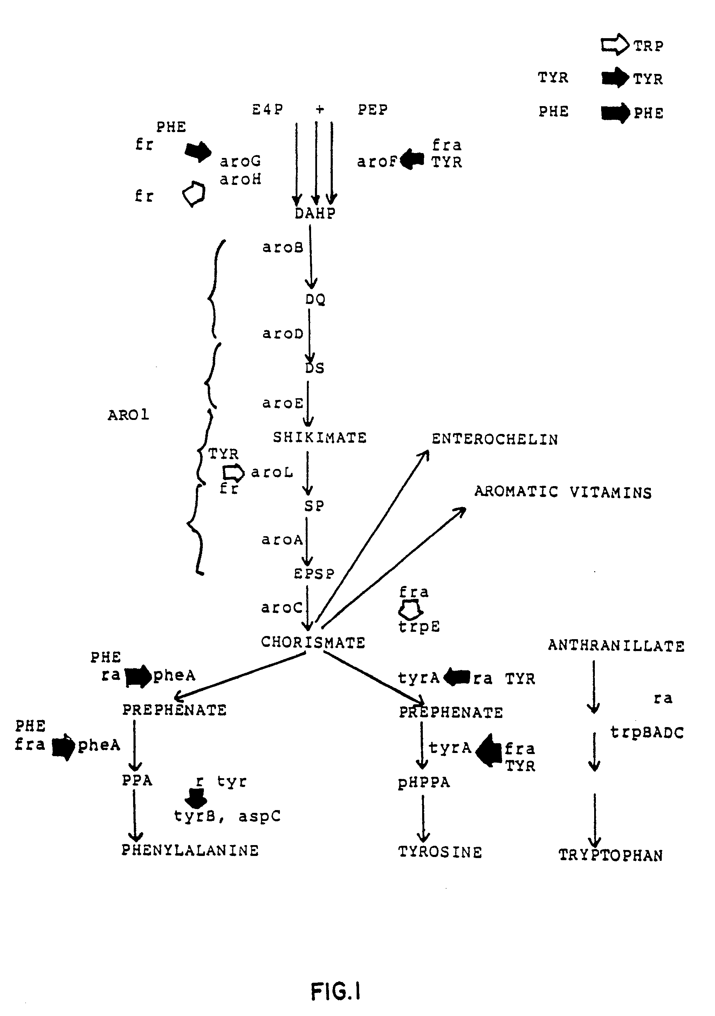

The biosynthesis of the aromatic amino acids is an example of a complex pathway that proceeds via a shared common pathway to chorismate where it branches to yield phenylalanine (ten steps), tyrosine (ten steps), tryptophan (twelve steps), the aromatic vitamins and enterochelins, see figure 1.

In E.coli (and other enteric organisms) the crucial step in the regulation of the common pathway is the first reaction catalyzed by the three DAHP synthetase isozymes. The rate of this reaction determines the rate at which carbon is committed to the pathway from the precursors phosphoenolpyruvate (PEP) and erythrose-4-phosphate (E4P). The three isozymes are each inhibited by one of the end products of aromatic amino acid biosynthesis and are coded for by the genes aroF (tyrosine sensitive) aroG (phenylalanine sensitive) and aroH (tryptophan sensitive). In the phenylalanine branch the regulated enzyme is chorismate mutaseprephenate dehydratase, the product of the pheA gene, a bifunctional enzyme in which the prephenate dehydratase activity is very sensitive to feedback inhibition by phenylalanine. Analogously in the tyrosine branch, the tyrA gene product chorismate mutase-prephenate dehydrogenase is sensitive to feedback inhibition by tyrosine.

In addition to feedback inhibition which is the main control operating to regulate carbon flux through the pathway, the levels of the above mentioned enzymes and shikimate kinase (aroL) are all subject to regulation by control at the transcriptional level. The pheA expression is controlled by the phenylalanine repressor, the product of the pheR gene, and by attenuation control. The aroF and tyrA genes together constitute a natural operon and their expression is coordinately regulated by the tyrosine repressor, the product of the tyrR gene. This tyrR also regulates the expression of aroG. Finally, the aroH gene expression is regulated by the tryptophan repressor, the product of the trpR gene.

As examples of amino acid production by composite plasmids we constructed plasmids that deregulate either phenylalanine or tyrosine production by suitably engineering the aroF, pheA and tyrA genes from E.coli K12. We used as a source of these genes a 6Kb EcoRI fragment isolated from a gene library by its ability to complement lesions in pheA and aroF G H. This fragment is essentially identical to that described by Zurawski et al. and was cloned into the multicopy vector pAT153 to yield pME65. The pheA and aroF genes were located and sequenced by established methods and deregulated as described in the Examples. Preferred composite plasmids and the host HW77 E.coli were deposited and are listed in the table of deposited plasmids.

## Table of Deposited Plasmids

| | |
|---|---|
| HW 77 (Host Cell) | ATCC 13281 |
| pME 202 | ATCC 53136 |
| pME 214 | ATCC 53137 |
| pME 219 | ATCC 53138 |
| pPT 112 | ATCC 53139 |

The American Type Culture Collection is located at 12301 Parktown Drive, Rockville, MD 20852, USA.

Example 1 – Preparation of PheA, AroF, AspC and Amylase Genes

1. Preparation of pheA gene

The sequence of the pheA gene is described in Chart 2. Insertion of a BamHI-BglII linker at the NcoI site was found to render the prephenate dehydratase activity of the enzyme substantially resistant to feed-back inhibition by L-phe. In addition, both the chorismate mutase and prephenate dehydratase activities of the enzyme were enhanced. Transcriptional deregulation was effected by replacing the promoter and attenuator sequences with a synthetic promoter based on the natural pheA promoter but lacking the dyad symmetry overlapping the Pribnow box sequence (−10). This replacement was made between the EcoRI site before the gene and a HaeII site lying in the N-terminus of the natural pheA gene. Finally, a BamHI site was introduced 3' to the pheA gene by directed mutagenesis. The resulting optimized pheA sequence is illustrated in Chart 3.

2. Preparation of aroF gene

The sequence of the aroF gene is described in Chart 4. A feedback inhibition resistant derivative was isolated in the following way : The aroF gene was first subcloned on a HindIII-BamHI fragment into the intermediate copy number vector pLG338. This was transformed into a strain (HW295) lacking all three DAHP synthetase isozymes. This resultant strain was then able to grow on minimal medium in the absence of exogenous aromatic amino acids and shikimate by virtue of the wild type aroF gene product carried on the plasmid. In the presence of 100 mcg tyrosine ml$^{-1}$, however, the aroF gene product is inhibited and consequently the strain is unable to synthesize sufficient Phe and Trp for growth. Mutants capable of growth on minimal media were isolated, pooled and retransform HW295 and the resulting transformants were screened for their ability to grow on minimal medium supplemented with tyrosine. Transformants capable of growth on this medium were assumed to have received a mutant plasmid and were

```
EcoRI
GAATTCACCAAGACGGGAAGACAAGAGGTAAAATTTATGACAATGAACATTACCAGCAAA  60
••••••
CAAATGGAAATTACTCCGGCCATCCGCCAACATGTCGCAGACCGTCTCGCCAAACTGGAA  120

AAATGGCAAACACATCTGATTAATCCACATATCATTCTGTCCAAAGAGCCACAAGGGTTT  180

GTTGCTGACGCCACAATCAATACACCTAACGGCGTTTCTGGTTGCCAGTGGTAAACATGAA  240

GATATGTACACCGCAATTAACGAATTGATCAACAAGCTGGAACGGCAGCTCAATAAACTG  300

CAGCACAAAGGCGAAGACGTCGTGCCGCAACATCGGTGAAAGACGCCAACTTCGTCGAAG  360

AAGTTGAAGAAGAGTAGTCCTTTATATTGAGTGTATCGCCAACGCGCCTTCGGGCGCGTT  420

TTTTGTTGACAGCGTGAAAACAGTACGGGTACTGTACTAAAGTCACTTAAGGAAACAAAC  480

                 M  K  H  I  P  P  P  P  A  P  P  P  P  T  P  P
ATGAAACACATACCGTTTTTCTTCGCATTCTTTTTTACCTTCCCCTGAATGGGAGGCGTT  540

TCGTCGTGTGAAACAGAATGCGAAGACGAACAATAAGGCCTCCCAAATCGGGGGGCCTTT  600

                          M  T  S  E  N  P  L  L  A  L  R
TTTATTGATAACAAAAAGGCAACACTATGACATCGGAAAACCCGTTACTGGCGCTGCGAG  660
                                             ↑↑↑↑↑↑
 E  K  I  S  A  L  D  E  K  L  L  A  L  L  A  E  R  R  E  L
AGAAAATCAGCGCGCTGGATGAAAAATTATTAGCGTTACTGGCAGAACGGCGCGAACTGG  720

 A  V  E  V  G  K  A  K  L  L  S  H  R  P  V  R  D  I  D  R
CCGTCGAGGTGGGAAAAGCCAAACTGCTCTCGCATCGCCCGGTACGTGATATTGATCGTG  780

 E  R  D  L  L  E  R  L  I  T  L  G  K  A  H  E  L  D  A  N
AACGGCGATTTGCTGGAAAGATTAATTACGCTCGGTAAAGCGCACCATCTGGACGCCCATT  840

 Y  I  T  R  L  P  Q  L  I  I  E  D  S  V  L  T  Q  Q  A  L
ACATTACTCGCCTGTTCCAGCTCATCATTGAAGATTCCGTATTAACTCAGCAGGCTTTGC  900

 L  Q  Q  H  L  N  K  I  N  P  H  S  A  R  I  A  F  L  G  P
TCCAACAACATCTCAATAAAATTAATCCGCACTCAGCACGCCATCGCTTTTCTCGGCCCCA  960

 K  G  S  Y  S  R  L  A  A  R  Q  Y  A  A  R  H  P  E  Q  P
AAGGTTCTTATTCCCATCTTGCGGCGCGCCAGTATGCTGCCCGGTCACTTTCAGCAATTCA  1020

 I  E  S  G  C  A  K  P  A  D  I  F  N  Q  V  E  T  G  Q  A
TTCAAAGTGGCTGCGCCAAATTTGCCGATATTTTTAATCAGGTGGAAACCGGCCAGGCGG  1080

 D  Y  A  V  V  P  I  E  H  T  S  S  G  A  I  H  D  V  Y  D
ACTATGCCGTCGTACCGATTGAAAAATACCAGCTCCGGTGCCATAAACGACGTTTACGATC  1140

 L  L  Q  H  T  S  L  S  I  V  G  E  H  T  L  T  I  D  H  C
TTCTGCAACATACCAGCTTGTCGATTGTTGGCGAGATGACGTTAACTATCGACCATTGTT  1200

 L  L  V  S  G  T  T  D  L  S  T  I  N  T  V  Y  S  H  P  Q
TGTTGGTCTCCGGCACTACTGATTTATCCACCATCAATACGGTCTACAGCCATCCGCAGC  1260

 P  P  Q  Q  C  S  K  P  L  N  R  Y  P  H  W  K  I  E  Y  T
CATTCCAGCAATGCAGCAAATTCCTTAATCGTTATCCGCACTGGAAGATTGAATATACCG  1320

 E  S  T  S  A  A  H  E  K  V  A  Q  A  K  S  P  H  V  A  A
AAAGTACGTCTGCGGCAATGGAAAAGGTTGCACAGGCAAAATCACCGCATGTTGCTGCGT  1380

 L  G  S  E  A  G  G  T  L  Y  G  L  Q  V  L  E  R  I  E  A
TGGGAAGCGAAGCTGGCGGCACTTTGTACGGTTTGCAGGTACTGGAGCGTATTGAAGCAA  1440

 H  Q  R  Q  N  P  T  R  P  V  V  L  A  R  K  A  I  N  V  S
ATCAGCGACAAAACTTCACCCGGATTTGTGGTGTTGGCGCGTAAAGCCATTAACGTGTCTG  1500

 D  Q  V  P  A  K  T  T  L  L  H  A  T  G  Q  Q  A  G  A  L
ATCAGGTTCCGGCGAAAACCACGTTGTTAATGGCGACCGGGCAACAAGCCGGTGCGCTGG  1560

 V  E  A  L  L  V  L  R  N  H  H  N  L  I  M  T  R  L  E  S  R
TTGAAGCGTTGCTGGTACTGCGCAACCACAATCTGATTATGACCCGTCTGGAATCACGCC  1620
```

```
     P   I   E   G   N   P   W   R   E   H   P   Y   L   D   I   Q   A   N   L   E
CGATTCACGGTAATCCATGGGAAGAGATGTTCTATCTGGATATTCAGGCCAATCTTGAAT 1686
             $$$$$$
   S   A   E   H   Q   K   A   L   K   E   L   G   E   I   T   R   S   H   K   V
CAGCGGAAATGCAAAAAGCATTGAAAGAGTTAGGGGAAATCACCCGTTCAATGAAGGTAT 1746

   L   G   C   Y   P   S   E   N   V   V   P   V   D   P   T   •••
TGGGCTGTTACCCAAGTGAGAACGTAGTGCCTGTTGATCCAACCTGATGAAAAGGTGCCG 1886

GATGATGTGAATCATCCGGCACTGGATTATTACTGGCGATTGTCATTCGCCTGACGCAAT 1866

AACACGCGGCTTTCACTCTGAAAACGCTGTGCGTAATCGCCGAACCAGTGCTCCACCTTG 1926

CGGAAACTGTCAATAAACGCCTGCTTATCGCCCTGCTCCAGCAACTCAATCGCCTCGCCG 1966

AAACGCTTATAGTAACGTTTGATTAACGCCAGATTACGCTCTGACGACATAATGATGTCG 2046

GCATAAAGCTGCGGATCC 2056
      ------
         BamHI
```

Chart 2. Sequence of the EcoRI-BamHI Fragment Encompassing
The phsA Gene.

KEY

The HaeII site used in the promoter swap          000000
The NcoI site used for the BglII linker mutagenesis   $$$$$$

```
       EcoRI      -35        PROMOTER      -18              SD   68
       GAATTCTTTTTTGTTGACAGCGTGAAAACAGTACGGGTATAATACTAAAGTCACAAAAAG
       ------   -------              ------              &&&

                M   T   S   E   N   P . L   L   A   L   R   E   K   I   S   A   L   128
       GCAACACTATGACATCGGAAAACCGTTACTGGCGCTGCGAGAGAAAATCAGCGCGCTGG
       &                                  !!!!!!!!
        D   E   K   L   L   A   L   L   A   E   R   R   E   L   A   V   E   V   G   K   188
       ATGAAAAATTATTAGCGTTACTGGCAGAACGGCGCGAACTGGCCGTCGAGGTGGGAAAAG

        A   K   L   L   S   H   R   P   V   R   D   I   D   R   E   R   D   L   L   E   248
       CCAAACTGCTCTCGCATCGCCCGGTACGTGATATTGATCGTGAACGCGATTTGCTGGAAA

        R   L   I   T   L   G   K   A   H   H   L   D   A   H   Y   I   T   R   L   F   308
       GATTAATTACGCTCGGTAAAGCGCACCATCTGGACGCCCATTACATTACTCGCCTGTTCC

        Q   L   I   I   E   D   S   V   L   T   Q   Q   A   L   L   Q   Q   H   L   N   368
       AGCTCATCATTGAAGATTCCGTATTAACTCAGCAGGCTTTGCTCCAACAACATCTCAATA

        K   I   N   P   H   S   A   R   I   A   P   L   G   P   K   G   S   Y   S   H   428
       AAATTAATCCGCACTCAGCACGCATCGCTTTTCTCGGCCCCAAAGGTTCTTATTCCCATC

        L   A   A   R   Q   Y   A   A   R   H   P   E   Q   P   I   E   S   G   C   A   488
       TTGCGGCGCGCCAGTATGCTGCCCGGTCACTTTGAGCAATTCATTGAAAGTGGCTGCGCCA

        K   P   A   D   I   F   N   Q   V   E   T   G   Q   A   D   Y   A   V   V   P   548
       AAATTTGCCGATATTTTTAATCAGGTGGAAACCGGCCAGGCCGACTATGCCGTCGTACCGA

        I   E   N   T   S   S   G   A   I   N   D   V   Y   D   L   L   Q   H   T   S   608
       TTGAAAATACCAGCTCCGGTGCCATAAACGACGTTTACGATCTGCTGCAACATACCAGCT

        L   S   I   V   G   E   M   T   L   T   I   D   H   C   L   L   V   S   G   T   668
       TGTCGATTGTTGGCGAGATGACGTTAACTATCGACCATTGTTTGTTGGTCTCCGGCACTA

        T   D   L   S   T   I   N   T . V   Y   S   H   P   Q   P   F   Q   Q   C   S   728
       CTGATTTATCCACCATCAATACGGTCTACAGCCATCCGCAGCCATTCCAGCAATGCAGCA

        K   P   L   N   R   Y   P   H   W   K   I   E   Y   T   E   S   T   S   A   A   788
       AATTCCTTAATCGTTATCCGCACTGGAAGATTGAATATACCGAAAGTACGTCTGCGGCAA

        M   E   K   V   A   Q   A   K   S   P   H   V   A   A   L   G   S   E   A   G   848
       TGGAAAAGGTTGCACAGGCAAAATCACCGCATGTTGCTGCGGTTGGGAAGCGAAGCTGGCG

        G   T   L   Y   G   L   Q   V   L   E   R   I   E   A   H   Q   R   Q   N   P   908
       GCACTTTGTACGGTTTGCAGGTACTGGAGCGTATTGAAGCAAATCAGCGACAAAACTTCA

        T   P   V   V   L   A   R   K   A   I   N   V   S   D   Q   V   P   A   K   968
       CCCGATTTGTCGGTGTTGGCGCGTAAAGCCATTAACGTGTCTGATCAGGTTCCGGCGAAAA

        T   T   L   L   M   A   T   G   Q   Q   A   G   A   L   V   E   A   L   L   V   1028
       CCACCGTTGTTAATGGCGACCGGGCAACAAGCCGGTGCGCTGGTTGAAGCGTTGCTGGTAC

        L   R   N   H   N   L   I   M   T   R   L   E   S   R   P   I   H   G   N   P   1088
       TGCGCAACCACAATCTGATTATGACCCGTCTGGAATCACGCCCGATTCACGGTAATCCAT

        W   R   S   P   W   E   E   M   F   Y   L   D   I   Q   A   N   L   E   S   A   1148
       GGAGATCTCCATGGGAAGAGATGTTCTATCTGGATATTCAGGCCAATCTTGAATCAGCGG
       --------------

        E   M   Q . K   A' L!  K   E   L   G   E   I   T   R   S   M   K   V   L   G   1208
       AAATGCAAAAAGCATTGAAAGAGTTAGGGGAAATCACCCGTTCAATGAAGGTATTGGGCT

        C   Y   P   S   E   N   V   V   P   V   D   P   T ******   1253
       GTTACCCAAGTGAGAACGTAGTGCCTGTTGATCCAACCTGATGAAAAGGATCC
                                                ------
                                                BamHI
```

Chart 3 Optimised pheA Sequence.

The sequence of the EcoRI-BamHI fragment carrying the pheA gene
from pHE198 is presented.

KEY

| The HaeII site used in the promoter swap | !!!!!!! |
| The 12 bp insertion conferring PBI-R | -------- |
| The Shine & Dalgarno sequence in the RBS. | &&&& |
| The -18 and -35 regions of the promoter | ------- |

HinDIII
AAGCTTTTACCGGAAAGTCCTCGGGCATAAGTAAAATCGGTCTCGGCAAGTTTTCTTTTGT 60
------

CCTTCGGCATATCCTTTAAGGTATAGACCGCGATCCGCCTGTGAATCGCTAAATGCATCG 120

TCATCTTTTATGGCGCTGCCCGATATCGCATCTTCCATACCGACATCATACCAGTCGGTG 180

CTGGTCAAGGTTGGCGCGTGAGTATAAGGATCAATCTGACATCCGCTAACCAGTAAAGCC 240

AACAAGGGGGCGATAAACTTTTTCATCATTTCTTTCTCCTTTTTCAAAGCATAGCGGATT 300

GTTTTCAAAGGGAGTGTAAATTTATCTATACAGAGGTAAGGGTTGAAAGCGCGACTAAAT 360

TGCCTGTGTAAATAAAAATGTACGAAATATGGATTGAAAACTTTACTTTATGTGTTATCG 420

<pre>
                                                        M
TTACGTCATCCTCGCTGAGGATCAACTATCGCAAACGAGCATAAACAGGATCGCCATCAT 480
                                              ^^^^^^
  Q  K  D  A  L  N  N  V  H  I  T  D  E  Q  V  L  M  T  P  E
GCAAAAAGACGCGCTGAATAACGTACATATTACCGACGAACAGGTTTTAATGACTCCGGA 540

  Q  L  K  A  A  F  P  L  S  L  Q  Q  E  A  Q  I  A  D  S  R
ACAACTGAAGGCCGCTTTTCCATTGAGCCTGCAACAAGAAGCCCAGATTGCTGACTCGCG 600

  R  S  I  S  D  I  I  A  G  R  D  P  R  L  L  V  V  C  G  P
TAAAAGCATTTCAGATATTATCGCCGGGCGCGATCCTCGTCTGCTGGTAGTATGTGGTCC 660

  C  S  I  H  D  P  E  T  A  L  E  Y  A  R  R  F  K  A  L  A
TTGTTCCATTCATGATCCGGAAACTGCTCTGGAATATGCTCGTCGATTTAAAGCCCTTGC 720

  A  E  V  S  D  S  L  Y  L  V  M  R  V  Y  P  E  K  P  R  T
CGCAGAGGTCAGCGATAGCCTCTATCTGGTAATGCGCGTCTATTTTGAAAAACCCCGTAC 780

  T  V  G  W  K  G  L  I  N  D  P  E  M  D  G  S  P  D  V  E
CACTGTCGGCTGGAAAGGGTTAATTAACGATCCCCATATGGATGGCTCTTTTGATGTAGA 840

  A  G  L  Q  I  A  R  K  L  L  L  E  L  V  N  M  G  L  P  L
AGCCGGGCTGCAGATCGCGCGTAAATTGCTGCTTGAGCTGGTGAATATGGGACTGCCACT 900

  A  T  E  A  L  D  P  N  S  P  Q  Y  L  G  D  L  P  S  W  S
GGCGACGGAAGCGTTAGATCCGAATAGCCCGCAATACCTGGGCGATCTGTTTAGCTGGTC 960

  A  I  G  A  R  T  T  E  S  Q  T  H  R  E  M  A  S  G  L  S
AGCAATTGGTGCTCGTACAACGGAATCGCAAACTCACCGTGAAATGGCCTCCGGGCTTTC 1020

  M  P  V  G  P  K  N  G  T  D  G  Q  L  A  T  A  I  N  A  M
CATGCCGGTTGGTTTTAAAAACGGCACCGACGGCAGTCTGGCAACAGCAATTAACGCTAT 1080

  R  A  A  A  Q  P  H  R  P  V  G  I  N  Q  A  G  Q  V  A  L
GCGCGCCGCCGCCCAGCCGCACCGTTTTGTTGGCATTAACCAGGCAGGGCAGGTTGCGTT 1140

  L  Q  T  Q  G  N  P  D  C  Q  V  I  L  R  G  G  K  A  P  N
GCTACAAACTCAGGGGAATCCGGACTGCCAGTGTGATCCGGCGCGGTGGTAAAGCGCCGAA 1200

  Y  S  P  A  D  V  A  Q  C  E  K  E  M  E  Q  A  G  L  R  P
CTATAGCCCTGCGGATGTTGCGCAATGTGAAAAAGAGATGGAACAGGCGGGACTGCGCCC 1260

  S  L  M  V  D  C  S  H  G  N  S  N  K  D  Y  R  R  Q  P  A
GTCTCTGATGGTAGATTGCAGCCACGGTAATTCGAAAAAGATTATCGCCGTCAGCCTGC 1320

  V  A  E  S  V  V  A  Q  I  K  D  G  N  R  S  I  I  G  L  M
GGTGGCAGAATCCGTGGTTGCTCAAATCAAAGATGGCAATCGCTCAATTATTGGTCTGAT 1380
</pre>

10

```
     I  E  S  M  I  E  E  G  N  Q  E  E  E  Q  P  R  E  E  N  E
   GATCGAAAGTAATATCCACGAGGGCAATCAGTCTTCCGAGCAACCGCGCAGTGAAATGAA  1448

     Y  G  V  S  V  T  D  A  C  I  S  W  E  N  T  D  A  L  L  R
   ATACGGTGTATCCGTAACCGATGCCTGCATTAGCTCGGAAATGACCGATGCCTTGCTGCG  1508

     E  I  N  Q  D  L  N  G  Q  L  T  A  R  V  A ***
   TGAAATTCATCAGGATCTGAAACGGGCAGCTGACGGCTCGCGTGGCTTAAGAGGTTTATTA  1568
                        iiiiii
   TGGTTGCTGAATTGACCGCATTACGCGATCAAATTGATGAAGTCGATAAAGCGCTGCTGA  1628

   ATTTATTAGCGAAGCGTCTGGAACTGGTTGCTGAAGTGGGCGAGGTGAAAAGCCGCTTTG  1688

   GACTGCCTATTTATGTTCCGGCAGCGCGAGGCATCTATGTTGGCCTCGCGTCGTGCAGAG  1748

   GCGGAAGCTCTGGGTGTACCGCCAGATCT  1769
                          ------
                          BglII
```

Chart 4    Sequence of the HindIII-BglII Fragment Encompassing
           the aroF Gene.

KEY

Sequence altered to a BamHI site in RBS                    ------.
Codon altered in FBI-R mutant (CAAasn-CGAarg)             .i.
PvuII site used in terminator swap underlined             iiiiii

assayed for DAHP synthetase activity. One mutant that carried a substantially feedback inhibition resistant aroF gene was kept for further analysis. Restriction fragment switching experiments revealed that the feedback lesion was located on a BstXI to PvuII fragment. Subsequent DNA sequencing revealed a single base pair alteration that changed glutamine 152 to an arginine residue.

The promoter and tyrR binding site were removed by introducing a BamHI site into the aroF ribosome binding site. Sequences downstream of the aroF gene were removed by replacing a PvuII-Sall fragment with a synthetic terminator also carried on a PvuII-Sall fragment. The sequence of the optimized aroF is illustrated in Chart 5.

In addition to pheA, aroF and tyrA genes, the aspC gene from E.coli K12 and the alpha amylase gene from B.licheniformis were prepared for use in composite plasmids.

3. Preparation of aspC gene

The cloning of the aspC gene has been described in European Patent Application 84100521.8 (Pub. No. 116860). For the purposes of the present invention it was necessary to sequence the gene and introduce convenient restriction sites. The DNA sequence is presented in Chart 6. A BamHI site was introduced into the ribosome binding site by directed mutagenesis. A BglII site was introduced downstream of the gene by making use of a convenient StuI site to insert a BglII linker. This enabled the aspC gene to be isolated on a convenient BamHI to BglII fragment. The optimized aspC gene is shown with ribosome binding site in Chart 7.

4. Preparation of Amylase gene

The cloning of the B.lichenoformis gene is described in European Patent Application 84308868.3. The nucleic acid sequence for the amylase gene is shown in Chart 8. Into the ribosome binding region we introduced a BamHI site by directed mutagenesis. A second BamHI site was introduced downstream of the gene by inserting BamHI linkers into a convenient HindIII site. Details of these modifications are described in Chart 9.

Examples of Composite plasmids and their applications

Example 2

Constructs in which the genes are transcriptionally deregulated by virtue of their high copy number.

The plasmid pME157 carries a FBI^R pheA gene transcribed from its wild type promoter cloned into the high copy number vector pAT153. The aroF gene along with a portion of the tyrA gene on a HindIII-BglII fragment was first modified by the addition of a BglII linker to the HindIII end and then cloned into the BamHI site of pME157. Two classes of plasmid resulted from this procedure. In the first (pME171) the pheA and aroF gene are transcribed colinearly. The second class (pME170) carries the two genes transcribed convergently. pME171 was then modified to encode a FBI^R aroF product by switching a BstXI fragment with the FBI^R aroF plasmid pMX13 to yield the deregulated plasmid pST35. The effect of these plasmids on the levels of DAHP synthetase and prephenate dehydratase are summarized in Table 1.

```
BamHI       M  Q  K  D  A  L  N  N  V  E  I  T  D  E  Q  V
GGATCCCCATCATGCAAAAAGACGCGCTGAATAACGTACATATTACCGACGAACAGGTTT 60
          ----

L  M  T  P  E  Q  L  K  A  A  P  P  L  S  L  Q  Q  E  A  Q
TAATGACTCCGGAACAACTGAAGGCCGCTTTTCCATTGAGCCTGCAACAAGAAGCCCAGA 120

I  A  D  S  R  K  S  I  S  D  I  I  A  G  R  D  P  R  L  L
TTGCTGACTCGCGTAAAAGCATTTCAGATATTATCGCCGGGCGCGATCCTCGTCTGCTGG 180

V  V  C  G  P  C  S  I  H  D  P  E  T  A  L  E  Y  A  R  R
TAGTATGTGGTCCTTGTTCCATTCATGATCCGGAAACTGCTCTGGAATATGCTCGTCGAT 240

P  K  A  L  A  A  E  V  S  D  S  L  Y  L  V  H  R  V  Y  P
TTAAAGCCCTTGCCGCAGAGGTCAGCGATAGCCTCTATCTGGTAATGCGGTCTATTTTTG 300

E  K  P  R  T  T  V  G  W  K  G  L  I  N  D  P  H  M  D  G
AAAAACCCCGTACCACTGTCGGCTGGAAAGGGTTAATTAACGATCCCCATATGGATGGCT 360

S  P  D  V  E  A  G  L  Q  I  A  R  K  L  L  L  E  L  V  N
CTTTTGATGTAGAAGCCGGGCTGCAGATCGCGCGTAAATTGCTGCTTGAGCTGGTGAATA 420

M  G  L  P  L  A  T  E  A  L  D  P  N  S  P  R  Y  L  G  D
TGGGACTGCCACTGGCGACGGAAGCGTTAGATCCGAATAGCCCGCGATACCTGGGCGATC 480

L  P  S  W  S  A  I  G  A  R  T  T  E  S  Q  T  H  R  E  M
TGTTTAGCTGGTCAGCAATTGGTGCTCGTACAACGGAATCGCAAACTCACCGTGAAATGG 540

A  S  G  L  S  M  P  V  G  F  K  N  G  T  D  G  S  L  A  T
CCTCCGGGCTTTCCATGCCGGTTGGTTTTAAAAACGGCACCGACGGCAGTCTGGCAACAG 600

A  I  N  A  H  R  A  A  A  Q  P  H  R  P  V  G  I  N  Q  A
CAATTAACGCTATGCGCGCCGCCGCCCAGCCGCACCGTTTTGTTCGGCATTAACCAGGCAG 660

G  Q  V  A  L  L  Q  T  Q  G  N  P  D  G  E  V  I  L  R  G
GGCAGGTTGCGTTGCTACAAACTCAGGGGAATCCGGACGGCCATGTGATCCTGCGCGGTG 720

G  K  A  P  N  Y  S  P  A  D  V  A  Q  C  E  K  E  M  E  Q
GTAAAGCGCCGAACTATAGCCCTGCGGATGTTGCGCAATGTGAAAAAGAGATGGAACAGG 780

A  G  L  R  P  S  L  M  V  D  C  S  H  G  N  S  N  K  D  Y
CGGGACTGCGCCCGTCTCTGATGGTAGATTGCAGCCACGGTAATTCCAATAAAGATTATC 840

R  R  Q  P  A  V  A  E  S  V  V  A  Q  I  K  D  G  N  R  S
GCCGTCAGCCTGCCGGTGGCAGAATCCGTGGTTGCTCAAATCAAAGATGGCAATCGCTCAA 900

I  I  G  L  M  I  E  S  N  I  H  E  G  N  Q  S  S  E  Q  P
TTATTGGTCTGATGATCGAAAGTAATATCCACGAGGGCAATCAGTCTTCCGAGCAACCGC 960

R  S  E  M  K  Y  G  V  S  V  T  D  A  C  I  S  W  E  M  T
GCAGTGAAATGAAATACGGTGTATCCGTAACCGATGCCTGCATTAGCTGGGAAATGACCG 1020

D  A  L  L  R  E  I  H  Q  D  L  N  G  Q  L  T  A  R  V  A *
ATGCCTTGCTGCGTGAAATTCATCAGGATCTGAACGGGCAGCTGACGGCTCGCGTGGCTT 1080

•••••••BamHI                              SalI
AATGAGGATCCCCGGGCCGATTCTACGCCCGGGGTTTTTTATGTCGAC 1125
      -----    SSSSSS        SSSSSS      -----
              SmaI            SmaI
```

**Chart 5** Optimised aroF Clone.

KEY

Site of PBI-R mutation                                    •|•
SmaI sites in terminator                               SSSSSS
Dyad symmetry in terminator
PvuII site used in terminator swap underlined          ••••••

```
TTCTAATAGCACACCTCTTTGTTAAATGCCGAAAAAACAGGACTTTCGTCCTGTTTTTT 60

TATACCTTCCAGAGCAATCTCACGTCTTGCAAAAACAGCCTGCGTTTTCATCAGTAATAG 120

TTGGAATTTTGTAAATCTCCCGTTACCCTGATAGCCGACTTCCCTTCTGTAACCATAATG 180

              M  F  N  I  T  A  A  P  A  D  F  I  L  G  L  A
GAACCTCGTCATGTTTGAGAACATTACCGCCGCTCCTGCCGACCCGATTCTGGGCCTGGC 240

  D  L  F  R  A  D  E  R  P  G  K  I  N  L  G  I  G  V  Y  K
CGATCTGTTTCGTGCCGATGAACGTCCCGGCAAAATTAACCTCGGGATTGGTGTCTATAA 300

  D  E  T  G  K  T  P  V  L  T  S  V  K  K  A  E  Q  Y  L  L
AGATGAGACGGGCAAAACCCCGGTACTGACCAGCGTGAAAAAGGCTGAACAGTATCTGCT 360

  E  N  T  T  K  N  Y  L  G  I  D  G  I  P  E  F  G  R  C
CGAAAATGAAACCACCAAAAATTACCTCGGCATTGACGGCATCCCTGAATTTGGTCGCTG 420

  T  Q  E  L  L  F  G  K  G  S  A  L  I  N  D  K  R  A  R  T
CACTCAGGAACTGCTGTTTGGTAAAGGTAGCGCCCTGATCAATGACAAACGTGCTCGCAC 480

  A  Q  T  F  G  G  T  G  A  L  R  V  A  A  D  F  L  A  K  N
GGCACAGACTCCGGGGGGCACTGGCGCACTACGCGTGGCTGCCGATTTCCTGGCAAAAA 540

  T  S  V  K  R  V  W  V  S  N  P  S  W  P  N  H  K  S  V  F
TACCAGCGTTAAGCGTGTGTGGGTGAGCAACCCAAGCTGGCCGAACCATAAGAGCGTCTT 600

  N  S  A  G  L  E  V  R  E  Y  A  Y  Y  D  A  E  N  H  T  L
TAACTCTGCAGGTCTGGAAGTTCGTGAATACGCTTATTATGATGCCGAAAATCACACTCT 660

  D  F  D  A  L  I  N  S  L  N  E  A  Q  A  G  D  V  V  L  F
TGACTTCGATGCACTGATTAACAGCCTGAATGAAGCTCAGGCTGGCGACGTAGTGCTGTT 720

  K  G  C  C  N  P  T  G  I  D  P  T  L  E  Q  W  Q  T  L
CCATGGCTGCTGCCATAACCCAACCGGTATCGACCCTACGCTGGAACAATGGCAAACACT 780

  A  Q  L  S  V  E  K  G  W  L  P  L  F  D  F  A  Y  Q  G  F
GGCACAACTCTCCGTTGAGAAAGGCTGGTTACCGCTGTTTGACTTCGCTTACCAGGGTTT 840

  A  R  G  L  E  E  D  A  E  G  L  R  A  F  A  A  N  H  K  E
TGCCCGTGGTCTCGAAGAAGATGCTGAAGGACTGCGCGCTTTCGCGGCTATGCATAAAGA 900

  L  I  V  A  S  S  Y  S  K  N  F  G  L  Y  N  E  R  V  G  A
GCTCATTGTTGCCAGTTCCTACTCTAAAAACTTTGGCCTGTACAACGAGCGTGTTGGCGC 960

  C  T  L  V  A  A  D  S  E  T  V  D  R  A  F  S  Q  H  K  A
TTGTACTCTGGTTGCTGCCGACAGTGAAACCGTTGATCGCGCATTCAGCCAAATGAAAGC 1020

  A  I  R  A  N  Y  S  N  F  F  A  H  G  A  S  V  V  A  T  I
GGCGATTCGCGCTAACTACTCTAACCCACCAGCACACGGCGCTTCTGTTGTTGCCACCAT 1080

  L  S  N  D  A  L  R  A  I  W  E  Q  E  L  T  D  N  R  Q  R
CCTGAGCAACGATGCCGTTACGTGCCATTTGGGAACAAGAGCTGACTGATATGCGCCAGCG 1140

  I  Q  R  N  R  Q  L  F  V  N  T  L  Q  E  K  G  A  N  R  D
TATTCAGCGTATGCGTCAGTTGTTCGTCAATACGCTGCAGGAAAAAGGCGCCAAACCGCGA 1200

  F  S  F  I  I  K  Q  N  G  N  F  S  F  S  G  L  T  K  E  Q
CTTCAGCTTTATCATCAAACAGAACGGCATGTTCTCCTTCAGTGGCCTGACAAAAGAACA 1260

  V  L  N  L  R  E  E  F  G  V  Y  A  V  A  S  G  R  V  N  V
AGTGCTGCGTCTGCGCGAAGAGTTTGGCGTATATGCCGGTTGCTTCTGGTCGCGTAAATGT 1320

  A  G  N  T  P  D  N  M  A  P  L  C  E  A  I  V  A  V  L  ...
GGCCGGGATGACACCAGATAACATGGCTCCGCTGTGCGAAGCGATTGTGGCAGTGCTGTA 1380

AGCATTAAAAACAATGAAGCCCGCTGAAAAGCGGGCTGAGACTGATCACAAACGCAACAT 1440

TGCCTGATGCGCTACGCTTATCAGGCCT 1466
```

Chart 6    Natural aroC Gene Sequence.

KEY

Site of BamHI site introduced in RBS                                ...·..

Table 1          DAHP Synthetase and Prephenate Dehydratase
                 Activities of HW77 Carrying Various Phe
                 Plasmids.

| PLASMID | PREPHENATE DEHYDRATASE | DAHP SYNTHASE |
|---------|------------------------|---------------|
| HW77 alone | ND | ND |
| pME214 | 19 | 65 |
| PME208 | 36 | 49 |
| pMH19 | 141 | 324 |
| pME202 | 705 | 1145 |
| pME219 | 491 | 694 |
| pME170 | 290 | 4505 |
| pME171 | 372 | 3570 |
| pST35 | 546 | 7433 |
| pME237 | 240 | 3146 |
| pME238 | 209 | 3420 |
| pAT153 | 3 | 34 |

HW77 was transformed with the plasmids indicated. Each strain was grown to $OD_{600}$ 1.0 in minimal medium supplemented with 0.5% casamino acids. Cell extracts were then prepared and used to assay the activities of prephenate dehydratase and DAHP synthase. Prephenate dehydratase activity was measured in the presence of L-phenylalanine. DAHP synthase activity was measured in the absence of feedback inhibitors and so represents the total DAHP synthase activity. Enzyme activities are expressed in mU min$^{-1}$ mg protein$^{-1}$.

The three plasmids pME171, pME170 and pST35 were introduced into the tyrA organism HW77. Cross-feeding experiments on these strains (Table 2) showed that the plasmids pME170 and pME171 caused a significant production of L-phenylalanine. HW77 carrying pST35, however, grew very badly on minimal medium and as a consequence showed very little cross-feeding. It appears that a high level of FBI$^R$ aroF is deleterious to the cell and that lower levels of the enzyme are required for optimum L-phenylalanine production under these conditions.

## Example 3

Composite plasmids which include a transcriptionally deregulated pheA gene.

The plasmid pJW2 carries the FBI$^R$ pheA gene transcribed from the synthetic promoter. Plasmid pME227 carries a FBI$^R$ aroF gene transcribed from its own promoter and ending with the synthetic terminator sequence described above. Both plasmids are based on the multicopy plasmid pAT153. An EcoRI fragment carrying pheA was constructed by the addition of EcoRI linkers to an EcoRI-BamHI fragment of pJW2 carrying pheA. This fragment was then cloned into the EcoRI site of pME227. The resulting plasmids carrying pheA transcribed in both orientations relative to aroF were designated pME238 (divergent) and pME237 (colinear). The DAHP synthetase and prephenate dehydratase activities in strains carrying these plasmids are given in Table 2.

Table 2 Cross-Feeding Experiments on HW77 Carrying Cloned Genes Pertaining to Phenylalanine Production (Phe Plasmids).

HW77 was transformed with all the plasmids described in the examples section to give the strains listed below. The relative extent of cross-feeding after 24 hr and 48 hr is indicated.

| PLASMID | CROSS-FEEDING OBSERVED | |
|---|---|---|
| | 24 hr | 48 hr |
| HW77 alone | (+) | + |
| pME214 | ++ | ++++ |
| PME208 | ++ | ++++ |
| pMH19 | +++ | +++++ |
| pME202 | +++ | +++++ |
| pME219 | ++ | ++++ |
| pME170 | ++ | ++++ |
| pME171 | ++ | ++++ |
| pST35 | NG | (+)* |
| pME237 | NG | (+)* |
| pME238 | NG | (+)* |
| pPT112 | ++ | +++ |
| pAT153 | (+) | + |

\* Some cross-feeding observed but from what were obviously a few mutant colonies that had managed to grow.

NG = no growth

Both plasmids when introduced into HW77 (ATCC 13281) prevented the strain from growing on minimal media. Again this is probably due to the excessive expression of FBI[R] _aroF_. As a result of this neither

```
BamHI      M  P  E  N  I  T  A  A  P  A  D  P  I  L  G  L
GGATCCTCGTCATGTTTGAGAACATTACCGCCGCTCCTGCCGACCCGATTCTGGGCCTGG  60

       A  D  L  P  R  A  D  E  R  P  G  K  I  N  L  G  I  G  V  Y
CCGATCTGTTTCGTGCCGATGAACGTCCCGGCAAAATTAACCTCGGGATTGGTGTCTATA  120

       K  D  E  T  G  K  T  P  V  L  T  S  V  K  K  A  E  Q  Y  L
AAGATGAGACGGGCAAAACCCCGGTACTGACCAGCGTGAAAAAGGCTGAACAGTATCTGC  180

       L  E  N  E  T  T  K  N  Y  L  G  I  D  G  I  P  E  F  G  R
TCGAAAATGAAACCACCAAAAATTACCTCGGCATTGACGGCATCCCTGAATTTGGTCGCT  240

       C  T  Q  E  L  L  P  G  K  G  S  A  L  I  N  D  K  R  A  R
GCACTCAGGAACTGCTGTTTGGTAAAGGTAGCGCCCTGATCAATGACAAACGTGCTCGCA  300

       T  A  Q  T  P  G  G  T  G  A  L  R  V  A  A  D  F  L  A  K
CGGCACAGACTCCGGGGGGCACTGGCGCACTACGCGTGGCTGCCGATTTCCTGGCAAAAA  360

       N  T  S  V  K  R  V  W  V  S  N  P  S  W  P  N  E  K  S  V
ATACCAGCGTTAAGCGTGTGTGGGTGAGCAACCCCAAGCTGGCCGAACCATAAGAGCGTCT  420

       F  N  S  A  G  L  E  V  R  E  Y  A  Y  Y  D  A  E  N  H  T
TTAACTCTGCAGGTCTGGAAGTTCGTGAATACGCTTATTATGATGCGGAAAATCACACTC  480

       L  D  F  D  A  L  I  N  S  L  N  E  A  Q  A  G  D  V  V  L
TTGACTTCGATGCACTGATTAACAGCCTGAATGAAGCTCAGGCTGGCGACGTAGTGCTGT  540

       F  H  G  C  C  H  N  P  T  G  I  D  P  T  L  E  Q  H  Q  T
TCCATGGCTGCTGCCATAACCCAACCGGTATCGACCCTACGCTGGAACAATGGCAAACAC  600

       L  A  Q  L  S  V  E  K  G  W  L  P  L  F  D  F  A  Y  Q  G
TGGCACAACTCTCCGTTGAGAAAGGCTGGTTACCGCTGTTTGACTTCGCTTACCAGGGTT  660

       F  A  R  G  L  E  E  D  A  E  G  L  R  A  F  A  A  N  H  K
TTGCCCGTGGTCTGGAAGAAGATGCTGAAGGACTGCGCGCTTTCGCGGCTATGCATAAAG  720

       E  L  I  V  A  S  S  Y  S  K  N  F  G  L  Y  N  E  R  V  G
AGCTGATTGTTGCCAGTTCCTACTCTAAAAACTTTGGCCTGTACAACGAGCGTGTTGGCG  780

       A  C  T  L  V  A  A  D  S  E  T  V  D  R  A  F  S  Q  H  K
CTTGTACTCTGGTTGCTGCCGACAGTGAAACCGTTGATCGCGCATTCAGCCAAATGAAAG  840

       A  A  I  R  A  N  Y  S  N  P  P  A  H  G  A  S  V  V  A  T
CGGCGATTCGCGCTAACTACTCTAACCCACCAGCACACGGCGCTTCTGTTGTTGCCACCA  900

       I  L  S  N  D  A  L  R  A  I  W  E  Q  E  L  T  D  M  R  Q
TCCTGAGCAACGATGCGTTACGTGCGATTTGGGAACAAGAGCTGACTGATATGCGCCAGC  960

       R  I  Q  R  M  R  Q  L  F  V  N  T  L  Q  E  K  G  A  N  R
GTATTCAGCGTATGCGTCAGTTGTTCGTCAATACGCTGCAGGAAAAAGGCGCCAAACCGCG  1020

       D  F  S  F  I  I  K  Q  N  G  M  F  S  F  S  G  L  T  F  F
ACTTCAGCTTTATCATCAAACAGAACGGCATGTTCTCCTTCAGTGGCCTGA.AAAAGAAC  1080

       Q  V  L  R  L  R  E  E  F  G  V  Y  A  V  A  S  G  R  V  N
AAGTGCTGCGTCTGCGCGAAGAGTTTGGCGTATATGCGGTTGCTTCTGGTCGCGTAAATG  1140

       V  A  G  M  T  P  D  N  M  A  P  L  C  E  A  I  V  A  V  L *
TGGCCGGGATCACACCAGATAACATGGCTCCGCTGTGCGAAGCGATTGTGGCAGTGCTGT  1200

       **
AAGCATTAAAAACAATGAAGCCCGCTGAAAAGCGGGCTGAGACTGATGACAAACGCAACA  1260
                                     BglII
TTGCCTGATGCGCTACGCTTATCAGGGAGATCT  1293
       ......
```

Chart 7 Optimised aspC Sequence.

EcoRI
GAATTCAGATTTTCTGAACAGGATTTCAAGCATTGCCTATCAGGAATTGAAAAATCGGAA 60

TCCGTCTGCTGACATGGCTTTATTAAAAGAAGGGACGAGCCCTCAGGAAGAACATTTCAG 120

CCGCTTTGCGCTTCTTGAAAACGAGGTGGAATTTTATTTTGAGAAAAAACAAACCGGTCT 180

TGAACAGTCTGTAAAAATAAAAAAAGAATGGGTAAAAGATATTTTAAAAGACCGATATCA 240

GGATATGAAAAAGAATCGTCTTCAGGCCAAACCTGATCAGGAGCCTGTTCCGCTTCCGAA 300

GCAAGCGAAAATTAATCCCGATGAAAAAGTGATTGCCCTCACATTTGATGACGGTCCGAA 360

TCCCGCTACAACGAATAAAATATTAAACGCTTTACAGAAGCATGAAGGGCATGCGACCTT 420

CTTTGTGCTTGGAAGCAGAGCCCAATATTATCCCGAAACGATAAAACGGATGCTGAAGGA 480

AGGAAACGAAGTCGGCAACCATTCCTGGGACCATCCGTTATTGACAAGGCTGTCAAACGA 540

AAAAGCGTATCAGGAGATTAACGACACGCAAGAAATGATCGAAAAAATCAGCGGACACCT 600

GCCTGTACACTTGCGTCCTCCATACGGCGGGATCAATGATTCCGTCCGCTCGCTTTCCAA 660

TCTGAAGGTTTCATTGTGGGATGTTGATCCGGAAGATTGGAAGTACAAAAATAAGCAAAA 720

GATTGTCAATCATGTCATGAGCCATGCGGGAGACGGAAAAATCGTCTTAATGCACGATAT 780

TTATGCAACGTCCGCAGATGCTGCTGAAGAGATTATTAAAAAGCTGAAAGCAAAAGGCTA 840

TCAATTGGTAACTGTATCTCAGCTTGAAGAAGTGAAGAAGCAGAGAGGCTATTGAATAAA 900

TGAGTAGAAAGCGCCATATCGGCGCTTTTCTTTTGGAAGAAAATATAGGGAAAATGGTAC 960

TTGTTAAAAATTCGGAATATTTATACAACATCATATGTTTCACATTGAAAGGGGAGGAGA 1020

```
         M  K  Q  Q  K  R  L  Y  A  R  L  L  T  L  L  F  A  L  I
ATCATGAAACAACAAAAACGGCTTTACGCCCGATTGCTGACGCTGTTATTTGCGCTCATC 1080

  F  L  L  P  H  S  A  A  A  A  A  N  L  N  G  T  L  M  Q  Y
TTCTTGCTGCCTCATTCTGCAGCAGCGGCGGCAAATCTTAATGGGACGCTGATGCAGTAT 1140

  F  E  W  Y  M  P  N  D  G  Q  H  W  K  R  L  Q  N  D  S  A
TTTGAATGGTACATGCCCAATGACGGCCAACATTGGAAGCGTTTGCAAAACGACTCGGCA 1200

  Y  L  A  E  H  G  I  T  A  V  W  I  P  P  A  Y  K  G  T  S
TATTTGGCTGAACACGGTATTACTGCCGTCTGGATTCCCCCCGGCATATAAGGGAACGAGC 1260

  Q  A  D  V  G  Y  G  A  Y  D  L  Y  D  L  G  E  F  H  Q  K
CAAGCGGATGTGGGCTACGGTGCTTACGACCTTTATGATTTAGGGGAGTTTCATCAAAAA 1320

  G  T  V  R  T  K  Y  G  T  K  G  E  L  Q  S  A  I  K  S  L
GGGACGGTTCGGACAAAGTACGGCACAAAGGAGAGCTGCAATCTGCGATCAAAGTCTT 1380

  H  S  R  D  I  N  V  Y  G  D  V  V  I  N  H  K  G  G  A  D
CATTCCCGCGACATTAACGTTTACGGGGATGTGGTCATCAACCACAAAGGCGGCGCTGAT 1440

  A  T  E  D  V  T  A  V  E  V  D  P  A  D  R  N  R  V  I  S
GCGACCGAAGATGTAACCGCGGTTGAAGTCGATCCCGCTGACCGCAACCGCGTAATTTCA 1500

  G  E  H  L  I  K  A  W  T  H  F  H  F  P  G  R  G  S  T  Y
GGAGAACACCTAATTAAAGCCTGGACACATTTTCATTTTCCCGGGGCGCGGCAGCACATAC 1560

  S  D  F  K  W  H  W  Y  H  F  D  G  T  D  W  D  E  S  R  K
AGCCGATTTTAAATGGCATTGGTACCATTTTGACGGAACCCGATTGGGACCGAGTCCCGAAAG 1620

  L  N  R  I  Y  K  F  Q  G  K  A  W  D  W  E  V  S  N  E  N
CTGAACCGCATCTATAAGTTTCAAGGAAAGGCTTGGGATTGGGAAGTTTCCAATGAAAAC 1680

  G  N  Y  D  Y  L  M  Y  A  D  I  D  Y  D  H  P  D  V  A  A
GGCAACTATGATTATTTGATGTATGCCGACATCGATTATGACCATCCTGATGTCGCAGCA 1740
```

```
  E  I  K  R  W  G  T  W  Y  A  N  E  L  Q  L  D  G  F  R  L
GAAATTAAGAGATGGGGCACTTGGTATGCCAATGAACTGCAATTGGACGGTTTCCGTCTT 1800

  D  A  V  K  N  I  K  F  S  F  L  R  D  W  V  N  H  V  R  E
GATGCTGTCAAACACATTAAATTTTCTTTTTTTGCGGGATTGGGTTAATCATGTCAGGGAA 1860

  K  T  G  K  E  M  F  T  V  A  E  Y  W  Q  M  D  L  G  A  L
AAAACGGGGAAGGAAATGTTTACGGTAGCTGAATATTGGCAGAATGACTTGGGCGCGCTG 1920

  E  M  Y  L  N  K  T  M  F  N  E  S  V  P  D  V  P  L  H  Y
GAAAACTATTTGAACAAAACAAATTTTAATCATTCAGTGTTTGACGTGCCGCTTCATTAT 1980

  Q  F  H  A  A  S  T  Q  G  G  G  Y  D  N  R  K  L  L  N  G
CAGTTCCATGCTGCATCGACACAGGGAGGCGGCTATGATATGAGGAAATTGCTGAACGGT 2040

  T  V  V  S  K  M  P  L  K  S  V  T  F  V  D  N  M  D  T  Q
ACGGTCGTTTCCAAGCATCCGTTGAAATCGGTTACATTTGTCGATAACCATGATACACAG 2100

  F  G  Q  S  L  E  S  T  V  Q  T  W  F  K  P  L  A  Y  A  F
CCCGGGCAATCGCTTGAGTCGACTGTCCAAACATGGTTTAAGCCGCTTGCTTACGCTTTT 2160

  I  L  T  R  E  S  G  Y  P  Q  V  F  Y  G  D  M  Y  G  T  K
ATTCTCACAAGGGAATCTGGATACCCTCAGGTTTTCTACGGGGATATGTACGGGACGAAA 2220

  G  D  S  Q  R  E  I  P  A  L  K  H  K  I  E  P  I  L  K  A
GGAGACTCCCAGCGCGAAATTCCTGCCTTGAAACACAAAATTGAACCGATCTTAAAAGCG 2280

  R  K  Q  Y  A  Y  G  A  Q  H  D  Y  F  D  H  H  D  I  V  G
AGAAAACAGTATGCGTACGGAGCACAGCATGATTATTTCGACCACCATGACATTGTCGGC 2340

  W  T  R  E  G  D  S  S  V  A  N  S  G  L  A  A  L  I  T  D
TGGACAAGGGAAGGCGACAGCTCGGTTGCAAATTCAGGTTTGGCGGCATTAATAACAGAC 2400

  G  P  G  G  A  K  R  M  Y  V  G  R  Q  N  A  G  E  T  W  H
GGACCCGGTGGGGCAAAGCGAATGTATGTCGGCCGGCAAAACGCCGGTGAGACATGGCAT 2460

  D  I  T  G  N  R  S  E  P  V  V  I  N  S  E  G  W  G  E  F
GACATTACCGGAAACCGTTCGGAGCCGGTTGTCATCAATTCGGAAGGCTGGGGAGAGTTT 2520

  H  V  N  G  G  S  V  S  I  Y  V  Q  R  ***
CACGTAAACGGCGGGTCGGTTTCAATTTATGTTCAAAGATAGAAGAGCAGAGAGGACGGA 2580

TTTCCTGAAGGAAATCCGTTTTTTTTATTTTGCCCGTCTTATAAATTTCTTTGATTACATT 2640

TTATAATTAATTTTAACAAAGTGTCATCAGCCCTCAGGAAGGACTTGCTGACAGTTTGAA 2700

TCGCATAGGTAAGGCGGGGATGAAATGGCAACGTTATCTGATGTAGCAAAGAAAGCAAAT 2760

GTGTCGAAAATGACGGTATCGCGGGTGATCAATCATCCTGAGACTGTGACGGATGAATTG 2820
  HindIII
AAAAAGCTT 2829
  ------
```

## Chart 8. Natural amylase gene sequence.

EP 0 229 161 B1

```
BamHI       M K Q Q K R L Y A R L L T L L F
GGATCCGAATCATGAAACAACAAAAACGGCTTTACGGCCCGATTGCTGACGGTGTTATTTG  60

A L I F L L P K S A A A A A N L M G T L
CGCTCATCTTCTTGCTGCCTCATTCTGCAGCAGCGGCGGCAAATCTTAATGGGACGCTGA  120

M Q Y F E W Y M P N D G Q M W K R L Q N
TGCAGTATTTTGAATGGTACATGCCCAATGACGGCCAACATTGGAAGCGTTTGCAAAACG  180

D S A Y L A E H G I T A V W I P P A Y K
ACTCGGCATATTTGGCTGAACACGGTATTACTGCCGTCTGGATTCCCCCGGCATATAAGG  240

G T S Q A D V G Y G A Y D L Y D L G E F
GAACGAGCCAAGCGGATGTGGGCTACGGTGCTTACGACCTTTATGATTTAGGGGAGTTTC  300

N Q K G T V R T K Y G T K G E L Q S A I
ATCAAAAAGGGACGGTTCGGACAAAGTACGGCACAAAAGGAGAGCTGCAATCTGCGATCA  360

K S L M S R D I N V Y G D V V I N H K G
AAAGTCTTCATTCCCGCGACATTAACGTTTACGGGGATGTGGTCATCAACCACAAAGGCG  420

G A D A T E D V T A V E V D P A D R N R
GCGCTGATGCGACCGAAGATGTAACCGCCGGTTGAAGTCGATCCCGCTGACCGCAACCGCG  480

V I S G E H L I K A W T H F H F P G R G
TAATTTCAGGAGAACACCTAATTAAAGCCTGGACACATTTTCATTTTCCGGGGCGCGGCA  540

S T Y S D F K W H W Y K F D G T D W D E
GCACATACAGCCATTTTAAATGGCATTGGTACCATTTTGACGGAACCGATTCGGACGGAGT  600

S R K L N R I Y K F Q G K A W D W E V S
CCCGAAAGCTGAACCGCATCTATAAGTTTCAAGGAAAGGCTTGGGATTGGGAAGTTTCCA  660

N E N G H Y D Y L M Y A D I D Y D K F D
ATGAAAACGGCAACTATGATTATTTGATGTATGCCGACATCGATTATGACCATCCTGATG  720

V A A E I K R W G T W Y A N E L Q L D G
TCGCAGCAGAAATTAAGAGATGGGGCACTTGGTATGCCAATGAACTGCAATTGGACGGTT  780

F R L D A V K H I K F S F L R D W V N M
TCCGTCTTGATGCTGTCAAACACATTAAAATTTTCTTTTTTGCGGGATTGGGTTAATCATG  840

V R E K T G K E M F T V A E Y W Q N D L
TCAGGGAAAAAACGGGGAAGGAAATGTTTACGGTAGCTGAATATTGGCAGAATGACTTGG  900

G A L E N Y L N K T N F N H S V F D V P
GCGGCGCTGGAAAACTATTTGAACAAACAAATTTTAATCATTCAGTGTTTGACGTGCCGC  960

L H Y Q F H A A S T Q G G G Y D M R K L
TTCATTATCAGTTCCATGCTGCATCGACACAGGGAGGCGGCTATGATATGAGGAAATTGC  1020

L N G T V V S K H P L K S V T F V D N M
TGAACGGTACGGTCGTTTCCAAGCATCCGTTGAAATCGGTTACATTTGTCGATAACCATG  1080

D T Q F G Q S L E S T V Q T W F K P L A
ATACACAGCCGGGGCAATCGCTTGAGTCGACTGTCCAAACATGGTTTAAGCCGCTTGCTT  1140

Y A F I L T R E S G Y P Q V F Y G D M Y
ACGGCTTTTATTCTCACAAGGGAATCTGGATACCCTCAGGTTTTCTACGGGGATATGTACG  1200

G T K G D S Q R E I P A L K H K I E F I
GGACGAAAGGAGACTCCCAGCGCCAAATTCCTGCCTTGAAACACAAAATTGAACCGATCT  1260

L K A R K Q Y A Y G A Q H D Y F D H H D
TAAAAGCCGAGAAAACAGTATGCGTACGGAGCACAGCATGATTATTTCGACCACCATCACA  1320

I V G W T R E G D S S V A N S G L A A L
TTGTCGGCTGGACAAGGGAAGGCGACAGCTCGGTTGCAAATTCAGGTTTGGCGGCATTAA  1380
```

```
    I   T   D   G   P   G   G   A   K   R   N   Y   V   G   R   Q   N   A   G   E
   TAACAGACGGACCCCGTGGGGCAAAGCGAATGTATGTCCGCCCGGCAAAACGCCGGTGAGA  1440

    T   W   N   D   I   T   G   N   R   S   E   P   V   V   I   N   S   E   G   W
   CATGGCATGACATTACCGGAAACCGTTCCGAGCCCGGTTGTCATCAATTCGGAAGGCTGGG  1500

    G   E   F   K   V   N   G   G   S   V   S   I   Y   V   Q   R  •••
   GAGAGTTTCACGTAAACGGCGGGTCGGTTTCAATTTATGTTCAAAGATAGAAGAGCAGAG  1560

   AGGACGGATTTCCTGAAGGAAATCCGTTTTTTTTATTTTGCCCGTCTTATAAATTTCTTTG  1620

   ATTACATTTTATAATTAATTTTAACAAAGTGTCATCAGCCCTCAGGAAGGACTTGCTGAC  1680

   AGTTTGAATCGCATAGGTAAGGCGCGGATGAAATGGCAACGTTATCTGATGTAGCAAACA  1740

   AAGCAAATGTGTCGAAAATCACCGTATCGCGCGTGATCAATCATCCTGAGACTGTGACGG  1800
                BamHI
   ATGAATTCAAAAAGCCTCCCGATCC  1824
                ••••••
```

Chart 9. Optimised amylase sequence.

strain gave significant zones of cross-feeding on minimal plates seeded with a phenylalanine auxotroph.

Example 4

A semi-synthetic operon construct.

In this example the pheA and aroF genes are combined into a single transcriptional unit. The plasmid pJW2 carries the pheA gene transcribed from the synthetic promoter. The mutant BamHI site downstream of pheA was used to clone the aroF gene carrying a BamHI site in its ribosome binding site. Finally, remaining tyrA sequence was removed by the addition of a synthetic terminator. This plasmid pME202 carries a semi-synthetic operon on an EcoRI-SalI fragment of 2, 400 bp. To further increase the utility of this fragment a derivative of pME202 was constructed that carries an EcoRI linker at the SalI site (pME204). Table 3 indicates the approximate numbers found. The plasmid pME202 (ATCC 53136) was introduced into a number of strains and the levels of DAHP synthetase and prephedate dehydratase determined during growth on a number of media. One example of these levels is indicated in Table 1. It is clear that the plasmid causes overproduction or the two activities and a comparison of enzyme levels during growth in the presence and absence of phenylalanine confirms that expression of the two genes has been substantially deregulated.

Table 3    Plasmid Constructs Using the Phe Operon from
           pME202 at various copy numbers.

```
-----------------------------------------------------------------------
          R1_P_****pheA****_****aroF****_T_S1 pME202

                              |
                              |  EcoRI linkers cloned
                              |  at SalI site
                              |
                              V
          R1_P_****pheA****_****aroF****_T_R1 pME204


     Phe operon now carried on EcoRI fragment "cas-
sette".  This fragment was recloned into the following
vectors:


-----------------------------------------------------------------------
pBR322 to give pMH19.      Copy number approximately  50.
pLG388 to give pME208.     Copy number approximately  10.
RP4 to give pME214.        Copy number approximately   2.
(pAT153 to give pME204).   Copy number approximately 100.
-----------------------------------------------------------------------
```

E.coli HW77 carrying pME202 was tested for L-phenylalanine production in cross-feeding experiments (Table 2). Extremely large zones were obtained in contrast to the untransformed controls. This demonstrates the advantage of being able to coordinate the expression of the two genes in the operon construct. The level of FBI$^R$ aroF product is now acceptable to HW77 growing on minimal medium.

### Example 5

Utility of the semi-synthetic phenylalanine operon construct in assessing the effect of varying plasmid copy number.

In order to ascertain the effect of copy number on expression the EcoRI fragment from pME204 that carried the aroF pheA operon was recloned into the following vectors : pBR322 (copy number 50) to give pMH19, pLG338 (copy number 10) to give pME208 and RP4 (copy number 1-3) to give pME214. These plasmids were introduced into the tyrA strains HW77 and HW760 and the resultant transformants tested for their growth on minimal medium and L-phe productivity in cross-feeding experiments. The results of these experiments are summarized in Table 4. It was found that while the high copy number derivatives were clearly deleterious to the growth of HW760, HW77 could tolerate all the plasmids. Furthermore, the lower copy derivatives pME208 and pME214 did not prevent the growth of HW760. HW760 carrying pME214 or pME208 gave very large zones of cross-feeding.

This example serves to indicate how the optimum expression level can be at calmed by varying the copy number of the pheA aroF operon through judicious choice of vector.

### Example 6

Extending the host range of the semi-synthetic phenylalanine operon.

The EcoRI fragment from pME204 that encompasses the pheA aroF operon was recloned into a number of broad host range vectors namely :

RP4 to give pME214
pCT460 to give pME211
pKT231 to give pME212

Table 4    Growth of HW77 and HW760 Carrying Various Phe
Plasmids.

| PLASMID | GROWTH | |
| --- | --- | --- |
| | 24 hr | 48 hr |
| − | ++ | ++++ |
| pME214 | ++ | ++++ |
| PME208 | ++ | ++++ |
| pMH19 | ++ | ++++ |
| pME202 | ++ | ++++ |
| pME219 | ++ | ++++ |
| pME170 | ++ | ++++ |
| pME171 | ++ | ++++ |
| pST35 | − | (+)* |
| pME237 | − | (+)* |
| pME238 | − | (+)* |
| pAT153 | ++ | ++++ |
| − | ++ | ++++ |
| pME214 | ++ | ++++ |
| pME208 | + | +++ |
| pMH19 | − | + |
| pME202 | − | (+) |

* Essentially no growth but a few mutant colonies
emerging.

The strains were grown overnight in L-broth supplemented with suitable antibiotic where appropriate. One microliter of the overnight culture was then streaked onto minimal agar supplemented with 50 ug ml$^{-1}$ tyrosine and any relevant antibiotic. Growth was scored after 24 hr and 48 hr.

These plasmids were then introduced by transformation, conjugation or mobilization into a variety of strains and L-phe production detected by cross-feeding of an E.coli L-phe auxotroph. Table 5 details the various organism/plasmid combinations and the cross-feeding observed. Significant cross-feeding was observed with a number of organisms including pseudomonads and facultative methylotrophs.

In principle this approach to deregulating a pathway by simply introducing such an artificial operon on a suitable plasmid could be extended to almost any microorganism that had beneficial characteristics. Expression would probably have to be reoptimized, e.g., for Gram positive organisms and this would entail alterations to the synthetic promoter and the various ribosome binding sites. Such an undertaking would be greatly facilitated by the modular structure of the operon and the presence of restriction sites in the ribosome binding sequences.

Example 7

Extension of the operor incorporation of the gene for alpha amylase.

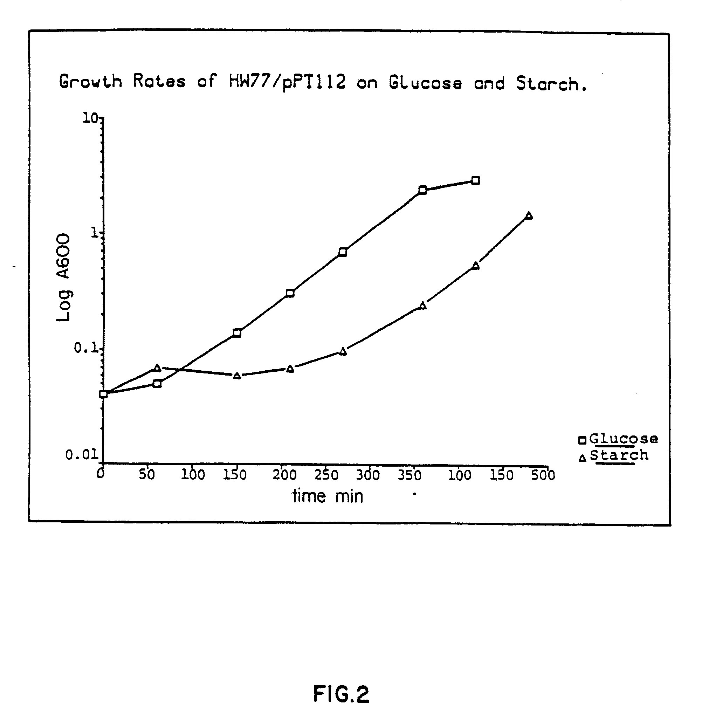

The modified alpha amylase gene is flanked by a BamHI site in the ribosome binding sequence and a BamHI linker in the HindIII site downstream of the gene. The BamHI fragment carrying amy was cloned into the downstream BamHI site in pME202 to give an artificial three gene operon (pPT112) deposited as ATCC 53139, see Chart 11. This plasmid was transformed into HW77 and its growth on starch investigated. A comparison of the growth of HW77/pPT112 on starch and glucose revealed that there was little difference between growth rates in starch and glucose, but growth on starch was subject to a much longer lag phase, see figure 2.

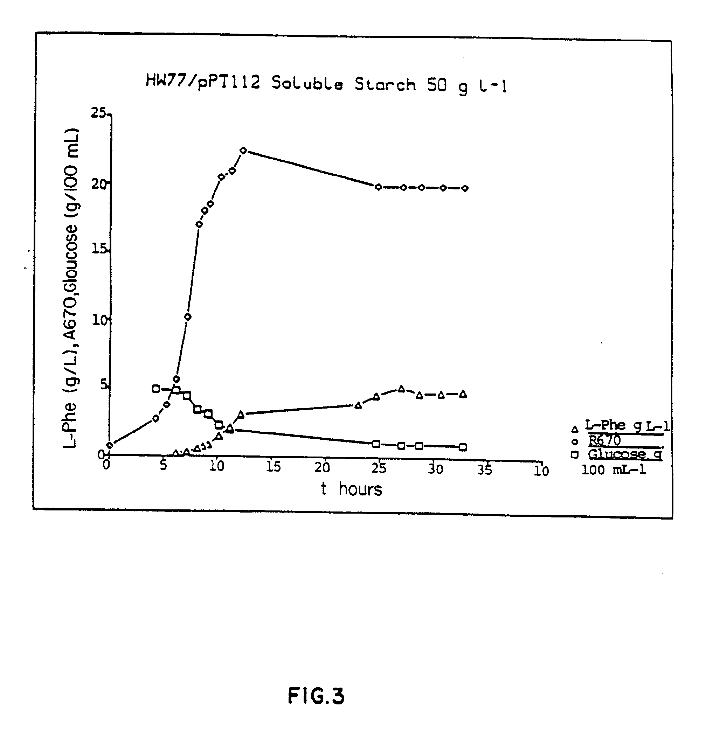

A fermentation performed on HW77/pPT112 grown on 50 g $1^{-1}$ soluble starch revealed significant accumulation of L-phe (figure 3). Fermentation conditions were as described in example 10 except that glucose was not included in the initial medium and a sugar feed was not used. The soluble starch was batched into the fermenter at 50 g $1^{-1}$ prior to autoclaving.

Table 5 Use of Phe Operon on broad host range plasmids to enhance L-phe production in other genera.
Plasmids : pME202 Phe operon in pAT153 – see text.

pME204 As pME204 but with EcoRI linker at SalI site.

pME211 Phe operon from pME204 cloned at EcoRI site of pCT460.

pME212 Phe operon from pME204 cloned at EcoRI site of pKT231, transcribed HindIII

pME213 Phe operon from pME204 cloned at EcoRI site of pKT231, transcribed str. Phe operon from pME204 cloned at EcoRI site of RP4, transcribed towards HindIII site.

| HOST | pME202 | pME214 | pME211 | pME212 | pME213 |
|---|---|---|---|---|---|
| E.coli | + | + | + | + | + |
| E.intermedia | − | | | | |
| S.marcesens | − | | | | |
| S.typhimurium | + | | | | |
| Ps.fluorescens | − | | + | + | + |
| Ps.putida | | | + | + | + |
| Facultative Methylotrophs: | (grown on methanol) | | | | |
| Mycoplana rubra | + | | | | |
| Pseudomonad spp. | + | | | | |
| Methylobacterium organophilum | − | | | | |
| Ps.extorquens | − | | | | |
| Vibrio exto.quens | − | | | | |

+ Production of L-Phe caused by introduction of plasmid as detected bv cross-feeding experiments or by TLC analysis of shake flask media.

− No L-Phe prduction detected in response to plasmid.

Remaining host/plasmid combinations were not tested.

Example 8

Extension of the operon – Incorporation of the aspC gene.

The artificial pheA aroF operon was further modified to include the gene for aspartate amino transferase (aspC) as follows : pIF18 which carries the aspC gene flanked by a BamHI site in the ribosome binding site and a BglII site downstream of the gene was used as a source of the BamHI-BglII fragment encompassing aspC. This fragment was then cloned into pME202 that had been partially cleaved with BamHI to give a derivative pME219 (deposited as ATCC 53138) that carries the aspC gene downstream of aroF so as to yield a three gene artificial operon (chart 10). When introduced into HW77 this plasmid caused L-phe production as detected by cross-feeding. Analysis of cell free extracts revealed elevated levels of aspC activity (Table 6).

Table 6    Phenotypic Effect of pME219.

pME219 was transformed into HW77 to give HW1058.

| Strain | Plasmid | Aspartate Aminotransferase Activity U $mg^{-1}$ |
|--------|---------|-------------------------------------------------|
| HW77   | –       | 604                                             |
| HW1058 | pME219  | 1913                                            |

The strains were grown to $O.D._{600}$ in glucose minimal medium supplemented with 0.5% casamino acids. The benefit of this construct is that the plasmid not only deregulates the phenylalanine pathway but also yields elevated aspC levels. This has utility in that an L-phe fermentation can be supplemented with chemically synthesized PPA along with aspartate or glutamate to increase L-phenylalanine titres. Such a hybrid fermentation/bioconversion has advantages that include greater ease of L-phe isolation and higher L-phe productivity.

```
                                                                      60
GAATTCTTTTTTGTTGACAGCGTGAAAACAGTACGGGTATAATACTAAAGTCACAAAAG   60

         M  T  S  E  M  P  L  L  A  L  R  E  E  I  S  A  L
GCAACACTATGACATCGGAAACCCGTTACTGGCGCTGCGAGAGAAATCAGCGCGCTGG  120

D  E  L  L  A  L  L  A  E  R  R  E  L  A  V  E  V  G  E
ATGAAAAATTATTAGCGTTACTGGCAGAACGGCGCGAACTGGCCGTCGAAGTGGGAAAAG  180

A  K  L  L  S  E  E  P  V  R  D  I  D  R  E  R  R  D  L  L  E
CCAAACTGCTCTCGCATCGCCCGGTACGTGATATTGATCGTGAACGCGATTTGCTCGAAA  240

R  L  I  T  L  G  R  A  E  E  L  D  A  E  Y  I  T  R  L  F
GATTAATTACGCTCGGTAAAGCGCACCATCTGGACGCCCATTACATTACTCGCCTGTTCC  300

Q  L  I  I  E  D  S  V  L  T  Q  Q  A  L  L  Q  Q  E  L  M
AGCTCATCATTGAAGATTCCGTATTAACTCAGCAGGCTTTGCTCCAACAACATCTCAATA  360

K  I  N  P  E  S  A  R  I  A  P  L  G  P  R  G  S  Y  E  E
AAATTAATCCGCACTCAGCACGCATCGCTTTTCTCGGCCCCAAAGGTTCTTATTCCCATC  420

L  A  A  R  Q  Y  A  A  R  E  F  R  Q  P  I  E  S  G  C  A
TTGCGGCGCGCCAGTATGCTGCCCCGTCACTTTGAGCAATTCATTGAAAGTGGCTGCGCCA  480

K  P  A  D  I  F  M  Q  V  E  T  G  Q  A  D  Y  A  V  V  P
AATTTGCCGATATTTTTAATCAGGTGGAAACCGGCCAGGCCGACTATGCCGTCGTACCGA  540

I  E  N  T  S  S  G  A  I  N  D  V  Y  D  L  L  Q  E  P  S
TTGAAAATACCAGCTCCGGTGCCATAAACGACGTTTACGATCTGCTGCAACATACCAGCT  600

L  S  I  V  G  E  M  T  L  T  I  D  E  C  L  L  V  S  G  T
TGTCGATTGTTGGCGAGATGACGTTAACTATCGACCATTGTTTGTTGGTCTCCGGCACTA  660

T  D  L  S  T  I  N  T  V  Y  S  E  P  Q  P  F  Q  Q  C  S
CTGATTTATCCACCATCAATACGGTCTACAGCCATCCGCAGCCATTCCAGCAATGCAGCA  720

R  F  L  N  R  Y  F  E  W  K  I  E  Y  T  E  S  T  S  A  A
AATTCCTTAATCGTTATCCGCACTGGAAGATTGAATATACCGAAAGTACGTCTGCGGCAA  780

M  E  K  V  A  Q  A  K  S  P  E  V  A  A  L  G  S  E  A  G
TGGAAAAGGTTGCACAGGCAAAATCACCGCATGTTGCTGCCGTTGGGAAGCGAAGCTGGCG  840

G  T  L  Y  G  L  Q  V  L  E  R  I  E  A  M  Q  R  Q  N  F
GCACTTTGTACGGTTTGCAGGTACTGGAGCGTATTGAAGCAAATCAGCGACAAAACTTCA  900

T  R  F  V  V  L  A  R  K  A  I  N  V  S  D  Q  V  P  A  K
CCCGATTTGTGGTGTTGGCGCGTAAAGCCATTAACGTGTCTGATCAGGTTCCGGCGAAAA  960

T  T  L  L  K  A  T  G  Q  Q  A  G  A  L  V  E  A  L  L  V
CCACGTTGTTAATGGCGACCGGGCAACAAGCCGGTGCGCTGGTTGAAGCGTTGCTGGTAC  1020

L  R  N  E  M  L  I  N  T  R  L  E  S  R  P  I  N  G  N  P
TGCGCAACCACAATCTGATTAATGACCCGTCTGGAATCACGCCCGATTCACGGTAATCCAT  1080

W  R  S  P  W  E  E  N  F  Y  L  D  I  Q  A  N  L  E  S  A
GGAGATCTCCATGGGAAGAGAATGTTCTATCTGGATATTCAGGCCAATCTTGAATCAGCGG  1140

E  M  Q  K  A  L  K  E  L  G  E  I  T  R  S  M  K  V  L  G
AAATGCAAAAAGCATTGAAAGAGTTAGGGGAAATCACCCGTTCAATGAAGGTATTGGGCT  1200

C  Y  P  S  E  M  V  V  P  V  D  P  T  ......          M
GTAACCCAAGTGAGAACGTAGTGCCTGTTGATCCAACCTGATGAAAAGGATCCCCATCAT  1260

   Q  K  D  A  L  M  M  V  E  I  T  D  E  Q  V  L  M  T  P  E
GCAAAAGACGCGCTGAATAACGTACATATTACCGACGAACAGGTTTTAATGACTCCGGA  1320

   Q  L  K  A  A  P  P  L  S  L  Q  Q  E  A  Q  I  A  D  S  R
ACAACTGAAGGCCGCTTTTCCATTGAGCCTGCAACAAGAAGCCCAGATTGCTGACTCGCG  1380

   K  S  I  S  D  I  I  A  G  R  D  P  R  L  L  V  V  C  G  P
TAAAAGCATTTCAGATATTATCGCCGGGCGCGATCCTCGTCTGCTGGTAGTATGTGGTCC  1440

   C  S  I  E  D  P  E  T  A  L  E  Y  A  R  R  F  K  A  L  A
TTGTTCCATTCATGATCCGGAAACTGCTCTGGAATATGCTCGTCGATTTAAAGCCCTTGC  1500

   A  E  V  S  D  S  L  Y  L  V  M  R  V  Y  Y  E  K  P  R  T
CGCAGACGGTCAGCCGATAGCCTCTATCTGGTAATGCGCGTCTATTTTGAAAAACCCCGTAC  1560
```

26

```
          T  V  G  W  E  G  L  I  N  D  P  E  N  D  G  S  F  D  V  E
CACTGTCGGCTGGAAAGGGTTAATTAACGATCCCCATATGGATGGCTCTTTTGATGTAGA 1620

          A  G  L  Q  I  A  E  E  L  L  L  E  L  V  N  N  G  L  P  L
AGCCGGGCTGCAGATCGCGCGTAAATTGCTGCTTGAGCTGGTGAATATGGGACTGCCACT 1680

          A  T  E  A  L  D  F  N  S  P  Q  Y  L  G  D  L  F  S  W  S
GGCGACGGAAGCGTTAGATCCGAATAGCCCGCAATACCTGGGCGATCTGTTTAGCTGGTC 1740

          A  I  G  A  R  T  T  E  S  Q  T  E  E  E  M  A  S  G  L  S
AGCAATTGGTGCTCGTACAACGGAATCGCAAACTCACCGTGAAATGGCCTCCGGGCTTTC 1800

          N  P  V  G  F  E  N  G  T  D  G  S  L  A  T  A  I  N  A  N
CATGCCGGTTGGTTTTAAAAACGGCACCGACGGCAGTCTGGCAACAGCAATTAACGCTAT 1860

          R  A  A  A  Q  P  E  E  F  V  G  I  N  Q  A  G  Q  V  A  L
GCGCGGCCGCCGCCCAGCCGCACCGTTTTGTTGGCATTAACCAGGCAGGGCAGGTTGCGTT 1920

          L  Q  T  Q  G  N  P  D  G  E  V  I  L  E  G  G  K  A  P  N
GCTACAAACTCAGGGGAATCCGGACGGCCATGTGATCCTGCGCGGTGGTAAAGCGCCGAA 1980

          Y  S  P  A  D  V  A  Q  C  E  K  E  N  E  Q  A  G  L  R  P
CTATAGCCCTGCGGATGTTGCGCAATGTGAAAAAGAGATGGAACAGGCGGGACTGCGCCC 2040

          S  L  N  V  D  C  S  E  G  N  S  N  K  D  Y  R  R  Q  P  A
GTCTCTGATGGTAGATTGCAGCCACGGTAATTCCAATAAAGATTATCGCCGTCAGCCTGC 2100

          V  A  E  S  V  V  A  Q  I  K  D  G  N  R  S  I  I  G  L  N
GGTGGCAGAATCCGTGGTTGCTCAAATCAAAGATGGCAATCGCTCAATTATTGGTCTGAT 2160

          I  E  S  N  I  E  E  G  N  Q  S  S  E  Q  P  R  S  E  N  K
GATCGAAAGTAATATCCACGAGGGCAATCAGTCTTCCGAGCAACCGCGCAGTGAAATGAA 2220

          Y  G  V  S  V  T  D  A  C  I  S  W  E  N  T  D  A  L  L  R
ATACGGTGTATCCGTAACCGATGCCTGCATTAGCTGGGAAATGACCGATGCCTTGCTGCG 2280

          E  I  N  Q  D  L  N  G  Q  L  T  A  R  V  A ******
TGAAATTCATCAGGATCTGAACGGGCAGCTGACGGCTCGCGTGGCTTAATGAGGATCCTC 2340

             M  F  E  N  I  T  A  A  P  A  D  P  I  L  G  L  A  D  L
GTCATGTTTGAGAACATTACCGCCGCTCCTGCCGACCCGATTCTGGGCCTGGCCGATCTG 2400

          F  R  A  D  E  R  P  G  K  I  N  L  G  I  G  V  Y  K  D  E
TTTCGTGCCGATGAACGTCCCGGCAAAATTAACCTCGGGATTGGTGTCTATAAAGATGAG 2460

          T  G  K  T  P  V  L  T  S  V  K  K  A  E  Q  Y  L  L  E  H
ACGGGCAAAACCCCGGTACTGACCAGCGTGAAAAAGGCTGAACAGTATCTGCTCGAAAAT 2520

          E  T  T  K  N  Y  L  G  I  D  G  I  P  E  F  G  R  C  T  Q
GAAACCACCAAAAAATTACCTCGGCATTGACGGCATCCCTGAATTTGGTCGCTGCACTCAG 2580

          E  L  L  F  G  K  G  S  A  L  I  N  D  K  R  A  R  T  A  Q
GAACTGCTGTTTGGTAAAGGTAGCGCCCTGATCAATGACAAACGTGCTCGCACGGCACAG 2640

          T  P  G  G  T  G  A  L  R  V  A  A  D  F  L  A  K  N  T  S
ACTCCGGGGGGGCACTGGCGCACTACGCGTGGCTGCCGATTTCCTGGCAAAAAAATACCAGC 2700

          V  K  R  V  W  V  S  N  P  S  W  P  N  H  K  S  V  P  N  S
GTTAAGCGTGTGTGGGGTGAGCAACCCAAGCTGGCCGAACCATAAGAGCGTCTTTAACTCT 2760

          A  G  L  E  V  R  E  Y  A  Y  Y  D  A  E  N  H  T  L  D  F
GCAGGTCTCGAAGTTCGTGAATACGCTTATTATGATGCGGAAAATCACACTCTTGACTTC 2820

          D  A  L  I  N  S  L  N  E  A  Q  A  G  D  V  V  L  F  E  G
GATGCACTGATTAACAGCCTGAATGAAGCTCAGGCTGGCGACGTAGTGCTGTTCCATGGC 2880

          C  C  E  N  F  T  G  I  D  P  T  L  E  Q  W  Q  T  L  A  Q
TGCTGCCATAACCCCAACCGGTATCGACCCTACGCTGGAACAATGGCAAACACTGGCACAA 2940

          L  S  V  E  K  G  W  L  P  L  F  D  F  A  Y  Q  G  F  A  R
CTCTCCGTTGAGAAAGGCTGGTTACCGCTGTTTGACTTCGCTTACCAGGGGTTTTGCCCGT 3000

          G  L  E  E  D  A  E  G  L  R  A  F  A  A  M  H  K  E  L  I
GGTCTGGAAGAAGATGCTGAAGGACTGCGCGCGTTTCGCGGCTATGCATAAAGAGCTGATT 3060

          V  A  S  S  Y  S  K  N  F  G  L  Y  N  E  R  V  G  A  C  T
GTTGCCAGTTCCTACTCTAAAAACTTTGGCCTGTACAACGAGCGTGTTGGCGCTTGTACT 3120
```

```
     L   V   A   A   D   S   E   T   V   D   R   A   F   S   Q   H   K   A   A   I
    CTGGTTGCTGCCGACAGTGAAACCGTTGATCGCGCATTCAGCCAAATGAAAGCGGCGATT 3180

     R   A   H   Y   S   H   P   P   A   H   G   A   S   V   V   A   T   I   L   S
    CGCGCTAACTACTCTAACCCACCAGCACACGGCGCTTCTGTTGTTGCCACCATCCTGAGC 3240

     H   D   A   L   R   A   I   W   E   Q   E   L   T   D   H   R   Q   R   I   Q
    AACGATGCGTTACGTGCGATTTGGGAACAAGAGCTGACTGATATGCGCCAGCGTATTCAG 3300

     R   M   R   Q   L   F   V   N   T   L   Q   E   K   G   A   N   R   D   F   S
    CGTATGCGTCAGTTGTTCGTCAATACGCTGCAGGAAAAAGGCGCAAACCGCGACTTCAGC 3360

     F   I   I   K   Q   N   G   R   F   S   F   S   G   L   T   K   E   Q   V   L
    TTTATCATCAAACAGAACGGCATGTTCTCCTTCAGTGGCCTGACAAAAGAACAAGTGCTG 3420

     R   L   R   E   E   F   G   V   Y   A   V   A   S   G   R   V   N   V   A   G
    CGTCTGCGCGAAGAGTTTGGCGTATATGCGGTTGCTTCTGGTCGCGTAAATGTGGCCGGG 3480

     M   T   P   D   N   H   A   P   L   C   E   A   I   V   A   V   L ***
    ATGACACCAGATAACATGGCTCCGCTGTGCGAAGCGATTGTGGCAGTGCTGTAAGCATTA 3540

    AAAACAATGAAGCCCGCTGAAAAGCGGGCTGAGACTGATGACAAACGCAACATTGCCTGA 3600

    TGCGCTACGCTTATCAGGGAGATCCCCGGCGATTCTACGCCCGGGTTTTTTTATGTCGAC 3659
                    X-----
```

Chart 10.    Sequence of the pheA aroF aspC
             operon in pME219.


KEY

Hybrid BglII/BamHI site downstream of aspC                    X-----

```
GAATTCTTTTTTGTTGACAGCCGTGAAAACAGTACGGGTATAATACTAAAGTCACAAAAG 60
          M  T  S  E  N  P  L  L  A  L  R  E  E  I  S  A  L  D
GCAACACTATGACATCGGAAAACCCGTTACTGGCGCTGCGAGAGAAAATCAGCGCGCTGG 120
   E  K  L  L  A  L  L  A  E  R  R  E  L  A  V  E  V  G  K  A
ATGAAAAATTATTAGCGTTACTGGCAGAACGGCGCGAACTGGCCGTCGAGGTGGGAAAAG 180
   K  L  L  S  M  R  P  V  E  D  I  D  R  E  R  D  L  L  E  R
CCAAACTGCTCTCGCATCGCCCGGTACGTGATATTGATCGTGAACGCGATTTGCTGGAAA 240
   L  I  T  L  G  K  A  M  M  L  D  A  H  Y  I  T  E  L  F  Q
GATTAATTACGCTCGGTAAAGCGCACCATCTGGACGCCCATTACATTACTCGCCTGTTCC 300
   L  I  I  E  D  S  V  L  T  Q  Q  A  L  L  Q  Q  E  L  N  K
AGCTCATCATTGAAGATTCCGTATTAACTCAGCAGGCTTTGCTCCAACAACATCTCAATA 360
   I  N  P  H  S  A  R  I  A  F  L  G  P  K  G  S  Y  S  H  L
AAATTAATCCGCACTCAGCACGCATCGCTTTTCTCGGCCCCAAAGGTTCTTATTCCCATC 420
   A  A  R  Q  Y  A  A  R  N  F  E  Q  F  I  E  S  G  C  A  K
TTGCGGCGCGCCAGTATGCTGCCCGTCACTTTGAGCAATTCATTGAAAGTGGCTGCGCCA 480
   F  A  D  I  F  N  Q  V  E  T  G  Q  A  D  Y  A  V  V  P  I
AATTTGCCGATATTTTTAATCAGGTGGAAACCGGCCAGGCCGACTATGCCGTCGTACCGA 540
   E  N  T  S  S  G  A  I  N  D  V  Y  D  L  L  Q  M  T  S  L
TTGAAAATACCAGCTCCGGTGCCATAAACGACGTTTACGATCTGCTGCAACATACCAGCT 600
   S  I  V  G  E  M  T  L  T  I  D  H  C  L  L  V  S  G  T  T
TGTCGATTGTTGGCGAGATGACGTTAACTATCGACCATTGTTTGTTGGTCTCCGGCACTA 660
   D  L  S  T  I  N  T  V  Y  S  H  P  Q  P  F  Q  Q  C  S  K
CTGATTTATCCACCATCAATACGGTCTACAGCCATCCGCAGCCATTCCAGCAATGCAGCA 720
   F  L  N  R  Y  P  H  W  K  I  E  Y  T  E  S  T  S  A  A  M
AATTCCTTAATCGTTATCCGCACTGGAAGATTGAATATACCGAAAGTACGTCTGCGGCAA 780
   E  K  V  A  Q  A  K  S  P  H  V  A  A  L  G  S  E  A  G  G
TGGAAAAGGTTGCACAGGCAAAATCACCGCATGTTGCTGCGTTGGGAAGCGAAGCTGGCG 840
   T  L  Y  G  L  Q  V  L  E  R  I  E  A  N  Q  R  Q  N  F  T
GCACTTTGTACGGTTTGCAGGTACTGGAGCGTATTGAAGCAAATCAGCGACAAAACTTCA 900
   R  P  V  V  L  A  R  K  A  I  N  V  S  D  Q  V  P  A  F  T
CCCGATTTGTGGTGTTGGCGCGTAAAGCCATTAACGTGTCTGATCAGGTTCCGGCGAAA 960
   T  L  L  M  A  T  G  Q  Q  A  G  A  L  V  E  A  L  L  V  L
CCACGTTGTTAATGGCGACCGGGCAACAAGCCGGTGCGCTGGTTGAAGCGTTGCTGGTAC 1020
   R  N  H  N  L  I  M  T  R  L  E  S  R  P  I  H  G  N  P  W
TGCGCAACCACAATCTGATTATGACCCGTCTGGAATCACGCCCGATTCACGGTAATCCAT 1080
   R  S  P  W  E  E  M  F  Y  L  D  I  Q  A  H  L  E  S  A  E
GGAGATCTCCATGGGAAGAGATGTTCTATCTGGATATTCAGGCCAATCTTGAATCAGCGG 1140
   M  Q  K  A  L  K  E  L  G  E  I  T  R  S  M  K  V  L  G  C
AAATGCAAAAAGCATTGAAAGAGTTAGGGGAAATCACCCGTTCAATGAAGGTATTGGGCT 1200
   Y  P  S  E  N  V  V  P  V  D  P  T  *              M
GTTACCCAAGTGAGAACGTAGTGCCTGTTGATCCAACCTGATGAAAAGGATCCCCATCAT 1260
   Q  K  D  A  L  N  N  V  H  I  T  D  E  Q  V  L  M  T  P  E
GCAAAAAGACGCGCTGAATAACGTACATATTACCGACGAACAGGTTTTAATGACTCCGGA 1320
   Q  L  K  A  A  F  P  L  S  L  Q  Q  E  A  Q  I  A  D  S  R
ACAACTGAAGGCCGCTTTTCCATTGAGCCTGCAACAAGAAGCCCAGATTGCTGACTCGCG 1380
   K  S  I  S  D  I  I  A  G  R  D  P  R  L  L  V  V  C  G  P
TAAAAGCATTTCAGATATTATCGCCGGGCGCGATCCTCGTCTGCTGGTAGTATGTGGTCC 1440
   C  S  I  H  D  P  E  T  A  L  E  Y  A  R  R  F  K  A  L  A
TTGTTCCATTCATGATCCCGAAACTGCTCTGGAATATGCTCGTCGATTTAAAGCCCTTGC 1500
   A  E  V  S  D  S  L  Y  L  V  M  R  V  Y  F  E  K  P  R  T
CGGCAGAGGTCAGCGGATAGCCTCTATCTGGTAATGCCGTCTATTTTGAAAAACCCCGTAC 1560
```

```
     T  V  G  W  K  G  L  I  N  D  P  H  H  D  G  S  P  D  V  E
CACTGTCGGCTGGAAAGGGTTAATTAACGATCCCCATATGGATGGCTCTTTTGATGTAGA 1620

     A  G  L  Q  I  A  R  K  L  L  L  E  L  V  N  N  G  L  P  L
AGCCGGGCTGCAGATCGCGCGTAAATTGCTGCTTGAGCTGGTGAATATGGGACTGCCACT 1680

     A  T  E  A  L  D  P  N  S  P  Q  Y  L  G  D  L  P  S  W  S
GGCGACGGAAGCGTTAGATCCGAATAGCCCGCAATACCTGGGCGATCTGTTTAGCTGGTC 1740

     A  I  G  A  R  T  T  E  S  Q  T  H  R  E  H  A  S  G  L  S
AGCAATTGGTGCTCGTACAACGGAATCGCAAACTCACCGTGAAATGGCCTCCGGGCTTTC 1800

     N  P  V  G  F  K  N  G  T  D  G  S  L  A  T  A  I  N  A  M
CATGCCGGTTGGTTTTAAAAACGGCACCGACGGCAGTCTGGCAACAGCAATTAACGCTAT 1860

     R  A  A  A  Q  P  H  R  F  V  G  I  N  Q  A  G  Q  V  A  L
GCGCGCCGCCGCCCAGCCGCACCGTTTTGTTGGCATTAACCAGGCAGGGCAGGTTGCGTT 1920

     L  Q  T  Q  G  N  P  D  G  N  V  I  L  R  G  G  K  A  P  N
GCTACAAACTCAGGGGAATCCGGACGGCCATGTGATCCTGCGCGGTGGTAAAGCGCCGAA 1980

     Y  S  P  A  D  V  A  Q  C  E  K  E  H  E  Q  A  G  L  R  P
CTATAGCCCTGCGGATGTTGCGCAATGTGAAAAAGAGATGGAACAGGCGGGACTGCGCCC 2040

     S  L  H  V  D  C  S  H  G  N  S  H  K  D  Y  R  R  Q  P  A
GTCTCTGATGGTAGATTGCAGCCACGGTAATTCCAATAAAGATTATCGCCGTCAGCCTGC 2100

     V  A  E  S  V  V  A  Q  I  K  D  G  N  R  S  I  I  G  L  M
GGTGGCAGAATCCGTGGTTGCTCAAATCAAAGATGGCAATCGCTCAATTATTGGTCTGAT 2160

     I  E  S  N  I  H  E  G  N  Q  S  S  E  Q  P  R  S  E  H  R
GATCGAAAGTAATATCCACGAGGGCAATCAGTCTTCCGAGCAACCGCGCAGTGAAATGAA 2220

     Y  G  V  S  V  T  D  A  C  I  S  W  E  H  T  D  A  L  L  R
ATACGGTGTATCCGTAACCGATGCCTGCATTAGCTGGGAAATGACCGATGCCTTGCTGCG 2280

     E  I  E  Q  D  L  N  G  Q  L  T  A  R  V  A  *  *
TGAAATTCATCAGGATCTGAACGGGCAGCTGACGGCTCGCGTGGCTTAATGAGGATCCGA 2340

     N  K  Q  Q  K  R  L  Y  A  R  L  L  L  T  L  L  P  A  L  I
ATCATGAAACAACAAAAACGGCTTTACGCCCGATTGCTGACGCTGTTATTTGCGCTCATC 2400

     P  L  L  P  N  S  A  A  A  A  A  N  L  N  G  T  L  H  Q  Y
TTCTTGCTGCCTCATTCTGCAGCAGCGGCGGCAAATCTTAATGGGACGCTGATGCAGTAT 2460

     P  E  W  Y  R  P  N  D  G  Q  H  W  K  R  L  Q  N  D  S  A
TTTGAATGGTACATGCCCAATGACGGCCAACATTGGAAGCGTTTGCAAAACGACTCGGCA 2520

     Y  L  A  E  H  G  I  T  A  V  W  I  P  P  A  Y  K  G  T  S
TATTTGGCTGAACACGGTATTACTGCCGTCTGGATTCCCCCGGCATATAAGGGAACGAGC 2580

     Q  A  D  V  G  Y  G  A  Y  D  L  Y  D  L  G  E  P  H  Q  K
CAAGCGGATGTGGGCTACGGTGCTTACGACCTTTATGATTTAGGGGAGTTTCATCAAAAA 2640

     G  T  V  R  T  K  Y  G  T  K  G  E  L  Q  S  A  I  K  S  L
GGGACGGTTCGGACAAAGTACGGCACAAAAGGAGAGCTGCAATCTGCGATCAAAAGTCTT 2700

     H  S  R  D  I  N  V  Y  G  D  V  V  I  N  H  K  G  G  A  D
CATTCCCGCGACATTAACGTTTACGGGGATGTGGTCATCAACCACAAAGGCGGCGCTGAT 2760

     A  T  E  D  V  T  A  V  E  V  D  P  A  D  R  N  K  V  I  S
GCGACCGAAGATGTAACCGCGGTTGAAGTCGATCCCGCTGACCGCAACCGCGTAATTTCA 2820

     G  E  H  L  I  K  A  W  T  H  P  H  P  P  G  R  G  S  T  Y
GGAGAACACCTAATTAAAGCCTGGACACATTTTCATTTTCCGGGGCGCGGCAGCACATAC 2880

     S  D  P  K  W  H  W  Y  H  P  D  G  T  D  W  D  E  S  R  K
AGCGATTTTAAATGGCATTGGTACCATTTTGACGGAACCGATTGGGACGAGTCCCGAAAG 2940

     L  N  R  I  Y  K  P  Q  G  K  A  W  D  W  E  V  S  N  E  N
CTGAACCGCATCTATAAGTTTCAAGGAAAGGCTTGGGATTGGGAAGTTTCCAATGAAAAC 3000

     G  N  Y  D  Y  L  M  Y  A  D  I  D  Y  D  H  P  D  V  A  A
GGCAACTATGATTATTTGATGTATGCCGACATCGATTATGACCATCCTGATGTCGCAGCA 3060

     E  I  K  R  W  G  T  W  Y  A  N  E  L  Q  L  D  G  F  R  L
GAAATTAAGAGATGGGGCACTTGGTATGCCAATGAACTGCAATTGGACGGTTTCCGTCTT 3120
```

30

```
D  A  V  R  I  I  K  F  S  F  L  R  D  W  V  N  H  V  R  E
GATGCTGTCAAACACATTAAATTTTCTTTTTTGCGGGATTGGGTTAATCATGTCAGGGAA  3180

K  T  G  K  E  H  F  T  V  A  E  Y  M  Q  N  D  L  G  A  L
AAAACGGGGAAGGAAATGTTTACGGTAGCTGAATATTGGCAGAATGACTTGGGCGCGCTG  3240

E  N  Y  L  N  K  T  N  F  N  H  S  V  F  D  V  P  L  H  Y
GAAAACTATTTGAACAAACAAATTTTAATCATTCAGTGTTTGACGTGCCGCTTCATTAT  3300

Q  F  H  A  A  S  T  Q  G  G  G  Y  D  H  R  K  L  L  N  G
CAGTTCCATGCTGCATCGACACAGGGAGGCGGCTATGATATGAGGAAATTGCTGAACGGT  3360

T  V  V  S  K  H  F  L  K  S  V  T  F  V  D  N  H  D  T  Q
ACGGTCGTTTCCAAGCATCCGTTGAAATCGGTTACATTTGTCGATAACCATGATACACAG  3420

F  G  Q  S  L  E  S  T  V  Q  T  W  F  K  P  L  A  Y  A  F
CCGGGGCAATCGCTTGAGTCGACTGTCCAAACATGGTTTAAGCCGCTTGCTTACGCTTTT  3480

I  L  T  R  E  S  G  Y  P  Q  V  F  Y  G  D  H  Y  G  T  K
ATTCTCACAAGGGAATCTGGATACCCTCAGGTTTTCTACGGGGATATGTACGGCACGAAA  3540

G  D  S  Q  R  E  I  P  A  L  K  H  K  I  E  P  I  L  K  A
GGAGACTCCCAGCGCGAAATTCCTGCCTTGAAACACAAAATTGAACCGATCTTAAAAGCG  3600

R  K  Q  Y  A  Y  G  A  Q  H  D  Y  F  D  H  H  D  I  V  G
AGAAAACAGTATGCGTACGGAGCACAGCATGATTATTTCGACCACCATGACATTGTCGGC  3660

W  T  R  E  G  D  S  S  V  A  H  S  G  L  A  A  L  I  T  D
TGGACAAGGGAAGGCGACAGCTCGGTTGCAAATTCAGGTTTGGCGGCATTAATAACAGAC  3720

G  P  G  G  A  K  R  H  Y  V  G  R  Q  N  A  G  E  T  W  H
GGACCCGGTGGGGCAAAGCGAATGTATGTCGGCCGGCAAAACGCCGGTCAGACATGGCAT  3780

D  I  T  G  N  R  S  E  P  V  V  I  N  S  E  G  W  G  E  F
GACATTACCGGAAACCGTTCGGAGCCGGTTGTCATCAATTCGGAAGGCTGGGGAGAGTTT  3840

H  V  N  G  S  V  S  I  Y  V  Q  R  *
CACGTAAACGGCGGGTCGGTTTCAATTTATGTTCAAAGATAGAAGAGCAGAGAGGACGGA  3900

TTTCCTGAAGGAAATCCGTTTTTTTTATTTTTGCCCGTCTTATAAATTTCTTTGATTACATT  3960

TTATAATTAATTTTAACAAAGTGTCATCAGCCCTCAGGAAGGACTTGCTGACAGTTTGAA  4020

TCGCATAGGTAAGGCGGGGATGAAATGGCAACGTTATCTGATGTAGCAAAGAAAGCAAAT  4080

GTGTCGAAAATGACGGTATCGCGGGTGATCAATCATCCTGAGACTGTGACGGATGAATTG  4160

AAAAAGCTCCGGATCCCGGGCGATTCTACGCCCGGGTTTTTTATGTCGAC  4150
```

Chart 11.  Sequence of the pheA aroF amy operon in pPT112.

## Example 9

Application of phenylalanine operon plasmids to strain screening.

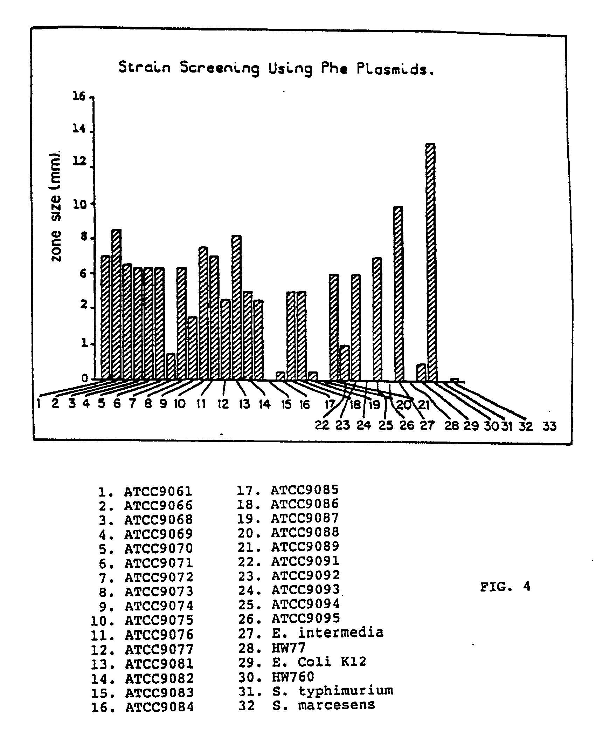

A total of 31 strains of E.coli, along with some other enteric species, were transformed with pME202 and screened for both growth on minimal medium and cross-feeding of an L-phe auxotroph. The results are summarized in figure 4. Since the complete deregulation of pheA and aroF would take 5-6 rounds of conventional mutagenesis it can be seen that the composite plasmid approach has a very great advantage in terms of speed. The mutagenesis of 45 strains to this extent would conservatively take ten man years to accomplish, and there would be no guarantee that the lesions would be directly comparable. Using the composite plasmid approach strains can easily be deregulated with identical lesions and put through initial screens in under two weeks.

## Example 10

Utility of Phe Plasmids in Fermentation.

To examine the potential of strains carrying Phe plasmids for L-phenylalanine production we compared the productivity of HW1057 (HW77pME202) with HW77 in 10 liter fermenters.

Both strains were grown from a single colony in L-broth for 15 hr. The HW1057 culture was supplemented with 100 µg ampicillin ml$^{-1}$. These overnight cultures were used to inoculate a shake flask containing the following minimal medium :

| Glucose | 19.4 |
|---|---|
| $(NH_4)_2HPO_4$ | 2.0 |
| $K_2PO_4$ | 6.6 |
| FeAmmCit | 0.1 |
| Tyrosine | 0.225 |
| $MgSO_4.7H_2O$ | 2.25 |

Rates of addition are given in grams per liter. Trace element solution was also added (1 ml per liter). Ampicillin was added to the medium for the growth of HW77pME202.

This seed culture was grown for 12 hr at 33° and then used to inoculate a 10 liter stirred tank fermenter containing the same medium. The fermentation was continued until the tyrosine was exhausted, i.e., about 11 hr at which point the glucose feed was commenced. Growth was limited by the tyrosine starvation to an $A_{670}$ of about 20. A 70% glucose feed was used at a rate of 0.63 g $1^{-1}$ $hr^{-1}$. L-phenylalanine concentrations were monitored throughout the fermentation.

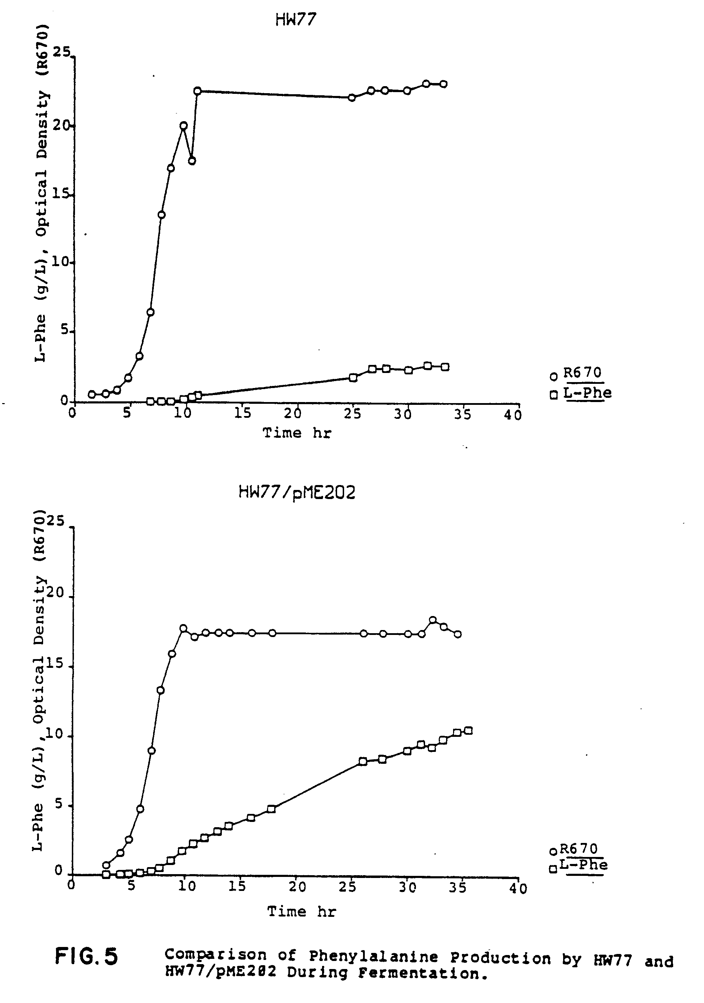

Figure 5 gives details of a typical comparison between HW77 and HW77 carrying the Phe plasmid pME202. It is clear that the presence of the plasmid has enhanced both the rate of accumulation and the final broth titre of L-phenylalanine. The details of productivity and final broth titre are summarized in the following table :

| STRAIN | PRODUCTIVITY (gm/Liter, hr) | FINAL TITRE (gm/Liter, hr) |
|---|---|---|
| HW77 | 0.11 | 6.07 |
| HW77+pME202 | 0.43 | 11.6 |

Analysis of the fermenter broths also revealed that whereas HW77 accumulated significant quantities of both prephenate and chorismate HW77pME202 did not. This indicates that the deregulated pheA gene on pME202 is having the desired effect of increasing the flux down the L-phenylalanine branch of the pathway by elevating prephenate dehydratase activity in vivo.

## Claims

1. A composite plasmid characterised in that it comprises a first DNA segment containing a replicon and a second DNA segment containing an artificial operon comprising two or more genes and a promoter the combination of which is not natively present in a single operon, the genes being selected from those mutant pheA, tyrA, aroF, aroG, aroH, aroL and trpE genes encoding enzymes which enzymes are feedback inhibition resistant and are active in aromatic amino acid synthesis.

2. A composite plasmid as claimed in claim 1 wherein at least one of the genes is a mutant pheA or aroF gene encoding a feedback inhibition resistant enzyme.

3. A composite plasmid as claimed in claim 1 or claim 2 wherein the artificial operon comprises an additional enzyme encoding gene selected from aroA, aroB, aroC, aroD, aroE, aroF, aroG, aroH, aroL, aspC, pheA, trpA, trpB, trpC, trpD, trpE, tyrA and tyrB genes encoding enzymes active in aromatic amino acid synthesis preferably aspC.

4. A composite plasmid as claimed in any of claims 1 to 3 wherein the artificial operon comprises an additional gene encoding one or more catabolic enzymes or transport proteins useful in amino acid production.

5. A composite plasmid as claimed in claim 4 wherein the additional gene encodes an amylase or permease protein.

6. A composite plasmid as claimed in any of claims 1 to 5 wherein the replicon is capable of replication in a prokaryotic cell.

7. A composite plasmid as claimed in any of claims 1 to 6 wherein it comprises plasmid pME202 (ATCC 53136) plasmid pME214 (ATCC 53137) plasmid pME219 (ATCC 53138) plasmid pPT112 (ATCC 53139) or plasmid pMH19.

8. A method of producing a composite plasmid as claimed in any of claims 1 to 7 characterised in that it comprises :

(a) mutating individually two or more genes selected from pheA, tyrA, aroF, aroG, aroH, aroL, and trE to encode feedback inhibition resistant enzymes active in aromatic amino acid synthesis ;

b) connecting the mutated genes into a transcriptional unit and c) expressing the transcriptional unit resulting in synthesis of feedback inhibition resistant enzymes thereby increasing the synthesis of the amino acid.

9. A method as claimed in claim 8 wherein an additional step comprises : inserting into the transcriptional unit one or more genes selected from aroA, aroB, aroC, aroD, aroE, aroF, aroG, aroH, aroL, aspC, pheA, trpA, trpB, trpC, trpD, trpE, tyrA and tyrB genes encoding enzymes active in aromatic amino acid synthesis ; and/or inserting into the transcriptional unit one or more genes encoding one or more catabolic enzymes or transport proteins useful in amino acid production.

10. A method of screening or selecting for microorganisms useful in amino acid production characterised in that it comprises :

(a) transforming a bacterial cell with a plasmid comprising a first DNA segment containing a replicon and a second DNA segment containing an artificial operon comprising two or more genes and a promoter the combination of which is not natively present in a single operon, the genes being selected from mutant pheA, tyrA, aroF, aroG, aroH, aroL, and trpE genes encoding feedback inhibition resistant enzymes which are normally rate-limiting in amino acid synthesis ;

(b) selecting the transformed cells ; .

(c) isolating a pure culture ;

(d) quantitating the production of the desired amino acid or an amino acid precursor from the pure culture of (c).

11. A method as claimed in claim 10 wherein an additional step comprises :

(e) cross-feeding to determine one or more of the following : (1) amino acid production, or (2) amino acid precursor production, and/or

(f) selection for growth in the presence of an amino acid analog.

12. A method for the production of an aromatic amino acid characterised in that it comprises : (a) fermentation using a bacterial cell culture containing a composite plasmid as claimed in any of claims 1 to 7 ; and (b) isolating the amino acid.

13. A method as claimed in claim 12 wherein the aromatic amino acid is phenylalanine tyrosine or tryptophan.

14. A composition of matter characterised in that it comprises a purified or isolated DNA sequence selected from the following :

(a) chart 3 ;

(b) chart 5 ;

(c) chart 7 ;

(d) chart 9 ;

(e) chart 10 ;

(F) chart 11.

15. A plasmid characterised in that it comprises a DNA sequence as claimed in claim 14.

16. A method for the production of an aromatic amino acid characterised in that it comprises :

(a) fermentation using a bacterial cell culture containing a plasmid as claimed in claim 15 ; and

(b) isolating the amino acid.

17. A method as claimed in claim 16 wherein the aromatic amino acid is phenylalanine, tyrosine or tryptophan.

18. An enzyme characterised in that it is active in the synthesis of phenylalanine is resistant to feedback inhibition by phenylalanine and has the amino acid sequence of the polypeptide given in chart 3 or is active in aromatic amino acid synthesis is resistant to feedback inhibition by tyrosine and has the amino acid sequence of the polypeptide given in chart 5.

**Ansprüche**

1. Kompositplasmid **dadurch gekennzeichnet,** daß es umfaßt ein erstes DNA-Segment, enthaltend ein Replikon, und ein zweites DNA-Segment, enthaltend ein künstliches Operon, umfassend zwei oder mehr Gene und einen Promotor, wobei deren Kombination nicht ursprünglich in einem einzigen Operon vorhanden ist, und wobei die Gene aus denjenigen mutierten pheA, tyrA, aroF, aroG, aroH, aroL und trpE Genen ausgewählt werden, die Enzyme codieren, welche feedback-inhibierungsresistent und bei der Synthese aromatischer Aminosäuren aktiv sind.

2. Kompositplasmid nach Anspruch 1, wobei wenigstens eines der Gene ein mutiertes pheA oder aroF Gen ist, welches ein feedback-inhibierungsresistentes Enzym codiert.

3. Kompositplasmid nach Anspruch 1 oder 2, wobei das künstliche Operon ein zusätzliches, Enzym codierendes Gen umfaßt, ausgewählt aus aroA, aroB, aroC, aroD, aroE, aroF, aroG, aroH, aroL, aspC, pheA, trpA, trpB, trpC, trpD, trpE, tyrA, und tyrB Genen, die Enzyme codieren, die bei der Synthese aromatischer Aminosäuren aktiv sind, bevorzugt aspC.

4. Kompositplasmid nach einem der Ansprüche 1-3, wobei das künstliche Operon ein zusätzliches Gen umfaßt, welches für eines oder mehrere katabolische Enzyme oder Transportproteine codiert, die bei der Aminosäureherstellung nützlich sind.

5. Kompositplasmid nach Anspruch 4, wobei das zusätzliche Gen ein Amylase- oder Permeaseprotein codiert.

6. Kompositplasmid nach einem der Ansprüche 1-5, wobei das Replikon zur Replikation in einer prokaryontischen Zelle fähig ist.

7. Kompositplasmid nach einem der Ansprüche 1-6, wobei es das Plasmid pME202 (ATCC 53136), Plasmid pME214 (ATCC 53137), Plasmid pME219 (ATCC 53138), Plasmid pPT112 (ATCC 53139) oder Plasmid pMH19 ist.

8. Verfahren zur Herstellung eines Kompositplasmids nach einem der Ansprüche 1-7, **dadurch gekennzeichnet,** daß es umfaßt :

a) einzelnes Mutieren zweier oder mehrerer Gene, ausgewählt aus pheA, tyrA, aroF, aroG, aroH, aroL und trpE, um feedback-inhibierungsresistente Enzyme zu codieren, die bei der Synthese aromatischer Aminosäuren aktiv sind ;

b) Verbinden der mutierten Gene zu einer Transkriptionseinheit und

c) Exprimieren der Transkriptionseinheit, wobei die Synthese feedback-inhibierungsresistenter Enzyme erfolgt, wodurch die Synthese der Aminosäure zunimmt.

9. Verfahren nach Anspruch 8, wobei ein zusätzlicher Schritt umfaßt :

Inserieren eines oder mehrerer Gene in die Transkriptionseinheit, ausgewählt aus aroA, aroB, aroC, aroD, aroE, aroF, aroG, aroH, aroL, aspC, pheA, trpA, trpB, trpC, trpD, trpE, tyrA und tyrB Genen, die Enzyme codieren, welche in der Synthese aromatischer Aminosäuren aktiv sind und/oder inserieren eines Gens oder mehrerer Gene, welche für eine oder mehreren katabolische Enzyme oder Transportproteine codieren, die in der Aminosäureproduktion nützlich sind, in die Transkriptionseinheit.

10. Verfahren zum Screenen oder Selektieren von bei der Aminosäureproduktion nützlichen Mikroorganismen, **dadurch gekennzeichnet,** daß es umfaßt :

a) Transformieren einer Bakterienzelle mit einem Plasmid, umfassend ein erstes DNA Segment, enthaltend ein Replikon, und ein zweites DNA Segment, enthaltend ein künstliches Operon, umfassend zwei oder meherere Gene und einen Promotor, wobei deren Kombination ursprünglich nicht in einem einzigen Operon vorliegt, und wobei die Gene aus mutierten pheA, tyrA, aroF, aroG, aroH, aroL und trpE-Genen ausgewählt werden, die feedback-inhibierungsresistente Enzyme codieren, die normalerweise in der Aminosäuresynthese geschwindigkeitslimitierend sind ;

b) Selektieren der transformierten Zellen ;

c) Isolieren einer Reinkultur ;

d) Quantifizierung der Produktion der gewünschten Aminosäure oder eines Aminosäurevorläufers aus der Reinkultur von (c).

11. Verfahren nach Anspruch 10, wobei ein zusätzlicher Schritt umfaßt :

(e) Kreuz-Feed, um einen oder mehrere der folgenden Parameter zu bestimmen : (1) Aminosäureproduktion oder (2) Aminosäurevorläuferproduktion und/oder

(f) Selektion auf Wachstum in Gegenwart eines Aminosäureanalogs.

12. Verfahren zur Herstellung einer aromatischen Aminosäure, dadurch gekennzeichnet, daß es umfaßt :

(a) Fermentation unter Verwendung einer Bakterienzellkultur, enthaltend ein Kompositplasmid nach einem der Asprüche 1-7 und

(b) Isolierung der Aminosäure.

13. Verfahren nach Anspruch 12, wobei die aromatische Aminosäure Phenylalanin, Tyrosin oder Tryptophan ist.

14. Stoffzusammensetzung, **dadurch gekennzeichnet,** daß sie umfaßt eine gereinigte oder isolierte DNA-Sequenz, ausgewählt aus der folgenden Sequenz :

(a) Tabelle 3 ;

(b) Tabelle 5 ;

(c) Tabelle 7 ;

(d) Tabelle 9 ;

(e) Tabelle 10 ;

(f) Tabelle 11.

15. Plasmid, dadurch gekennzeichnet, daß es eine DNA-Sequenz nach Anspruch 14 umfaßt.

16. Verfahren zur Herstellung einer aromatischen Aminosäure, dadurch gekennzeichnet, daß es umfaßt :

(a) Fermentation unter Verwendung einer Bakterienzellkultur, enthaltend ein Plasmid nach Anspruch 15 und

(b) Isolierung der Aminosäure.

17. Verfahren nach Anspruch 16, wobei die aromatische Aminosäure Phenylalanin, Tyrosin oder Tryptophan ist.

18. Enzym, dadurch gekennzeichnet, daß es in der Synthese von Phenylalanin aktiv ist, gegen die Feedback-Inhibierung durch Phenylalanin resistent ist und es die Aminosäuresequenz des Polypeptids in Tabelle 3 aufweist oder es in der aromatischen Aminosäuresynthese aktiv ist, gegen die Feedback-Inhibierung durch Tyrosin resistent ist und es die Aminosäuresequenz des Polypeptids aus Tabelle 5 aufweist.


## Revendications

1. Plasmide composite, caractérisé par le fait qu'il comprend un premier segment d'ADN contenant un réplicon et un second segment d'ADN contenant un opéron artificiel comprenant au moins deux gènes et un promoteur, dont la combinaison n'est pas présente de façon native dans un opéron simple, les gènes étant choisis parmi les gènes mutants pheA, tyrA, aroF, aroG, aroH, aroL et trpE codant pour des enzymes, lesquelles enzymes sont résistantes à la rétro-inhibition et sont actives dans la synthèse d'acides aminés aromatiques.

2. Plasmide composite selon la revendication 1, dans lequel au moins l'un des gènes est un gène mutant pheA ou aroF codant pour une enzyme résistante à la rétro-inhibition.

3. Plasmide composite selon la revendication 1 ou la revendication 2, dans lequel l'opéron artificiel comprend un gène codant pour une enzyme supplémentaire, choisi parmi les gènes aroA, aroB, aroC, aroD, aroE, aroF, aroG, aroH, aroL, aspC, pheA, trpA, trpB, trpC, trpD, trpE, tyrA et tyrB codant pour des enzymes actives dans la synthèse d'acides aminés aromatiques, de préférence, asPC.

4. Plasmide composite selon l'une des revendications 1 à 3, dans lequel l'opéron artificiel comprend un gène supplémentaire codant pour au moins une enzyme catabolique ou protéine de transport utile dans la production d'acides aminés.

5. Plasmide composite selon la revendication 4, dans lequel le gène supplémentaire code pour une protéine de type amylase ou perméase.

6. Plasmide composite selon l'une des revendications 1 à 5, dans lequel le réplicon est capable de réplication dans une cellule procaryote.

7. Plasmide composite selon l'une des revendications 1 à 6, qui comprend le plasmide pME202 (ATCC 53136), le plasmide pME214 (ATCC 53137), le plasmide pME219 (ATCC 53138), le plasmide pPT112 (ATCC 53139) ou le plasmide pMH19.

8. Procédé de production d'un plasmide composite tel que défini dans l'une des revendications 1 à 7, caractérisé par le fait qu'il comprend :

(a) la mutation individuelle d'au moins deux gènes choisis parmi pheA, tyrA, aroF, aroG, aroH, aroL et trpE pour coder pour des enzymes résistantes à la rétro-inhibition, actives dans la synthèse d'acides aminés aromatiques ;

(b) la connexion des gènes ayant subi une mutation dans une unité de transcription ; et

(c) l'expression de l'unité de transcription conduisant à la synthèse d'enzymes résistantes à la rétro-inhibition, accroissant ainsi la synthèse de l'acide aminé.

9. Procédé selon la revendication 8, dans lequel une étape supplémentaire comprend :

– l'insertion dans l'unité de transcription d'au moins un gène choisi parmi les gènes aroA, aroB, aroC, aroD, aroE, aroF, aroG, aroH, aroL, aspC, pheA, trpA, trpB, trpC, trpD, trpE, tyrA et tyrB codant pour des enzymes actives dans la synthèse d'acides aminés aromatiques ; et/ou

– l'insertion dans l'unité de transcription d'au moins un gène codant pour au moins une enzyme catabolique ou protéine de transport utile dans la production d'acides aminés.

10. Procédé de criblage ou de sélection de micro-organismes utiles dans la production d'acides aminés, caractérisé par le fait qu'il comprend :

(a) la transformation d'une cellule bactérienne par un plasmide comprenant un premier segment d'ADN et contenant un réplicon et un second segment d'ADN contenant un opéron artificiel comprenant au moins deux gènes et un promoteur, dont la combinaison n'est pas présente de façon native dans un opéron simple, les gènes étant choisis parmi les gènes mutants pheA, tyrA, aroF, aroG, aroH, aroL et trE codant pour des enzymes résistantes à la rétro-inhibition, qui sont normalement limitantes du rendement dans la synthèse d'acides aminés ;

(b) la sélection des cellules transformées ;

(c) l'isolement d'une culture pure ;

(d) la quantification de la production de l'acide aminé désiré ou d'un précurseur de l'acide aminé désiré à partir de la culture pure de (c).

11. Procédé selon la revendication 10, dans lequel une étape supplémentaire comprend :

(e) le cross-feeding pour déterminer au moins l'un des points suivants : (1) la production d'acide aminé, ou (2) la production de précurseur d'acide aminé ; et/ou

(f) la sélection pour la croissance en présence d'un analogue d'acide aminé.

12. Procédé de production d'un acide aminé aromatique,caractérisé par le fait qu'il comprend :

(a) une fermentation à l'aide d'une culture de cellules bactériennes contenant un plasmide composite tel que défini à l'une des revendications 1 à 7 ; et

(b) l'isolement de l'acide aminé.

13. Procédé selon la revendication 12, dans lequel l'acide aminé aromatique est la phénylalanine, la tyrosine ou le tryptophane.

14. Composition de matière,caractérisée par le fait qu'elle comprend une séquence d'ADN purifiée ou isolée, choisie parmi les suivantes :

(a) Diagramme 3 ;

(b) Diagramme 5 ;

(c) Diagramme 7 ;

(d) Diagramme 9 ;

(e) Diagramme 10 ;

(f) Diagramme 11 ;

15. Plasmide, caractérisé par le fait qu'il comprend une séquence d'ADN telle que définie à la revendication 14.

16. Procédé de production d'un acide aminé aromatique,caractérisé par le fait qu'il comprend :

(a) une fermentation à l'aide d'une culture de cellule bactérienne contenant un plasmide tel que défini à la revendication 15 ; et

(b) l'isolement de l'acide aminé.

17. Procédé selon la revendication 16, dans lequel l'acide aminé aromatique est la phénylalanine, la tyrosine ou le tryptophane.

18. Enzyme,caractérisée par le fait qu'elle est active dans la synthèse de la phénylalanine, qu'elle est résistante à la rétro-inhibition par la phénylalanine et qu'elle présente la séquence d'acides aminés du polypeptide donné dans le Diagramme 3 ou qu'elle est active dans la synthèse d'acides aminés aromatiques, qu'elle est résistante à la rétro-inhibition par la tyrosine et qu'elle présente la séquence d'acides aminés du polypeptide donné dans le Diagramme 5.

FIG.1

Growth Rates of HW77/pPT112 on Glucose and Starch.

FIG.2

38

HW77/pPT112 Soluble Starch 50 g L-1

FIG.3

FIG. 4

| 1. | ATCC9061 | 17. | ATCC9085 |
|----|----------|-----|----------|
| 2. | ATCC9066 | 18. | ATCC9086 |
| 3. | ATCC9068 | 19. | ATCC9087 |
| 4. | ATCC9069 | 20. | ATCC9088 |
| 5. | ATCC9070 | 21. | ATCC9089 |
| 6. | ATCC9071 | 22. | ATCC9091 |
| 7. | ATCC9072 | 23. | ATCC9092 |
| 8. | ATCC9073 | 24. | ATCC9093 |
| 9. | ATCC9074 | 25. | ATCC9094 |
| 10. | ATCC9075 | 26. | ATCC9095 |
| 11. | ATCC9076 | 27. | E. intermedia |
| 12. | ATCC9077 | 28. | HW77 |
| 13. | ATCC9081 | 29. | E. Coli K12 |
| 14. | ATCC9082 | 30. | HW760 |
| 15. | ATCC9083 | 31. | S. typhimurium |
| 16. | ATCC9084 | 32 | S. marcesens |

40

HW77

HW77/pME202

FIG. 5    Comparison of Phenylalanine Production by HW77 and HW77/pME202 During Fermentation.